# EUROPEAN PATENT SPECIFICATION

(11) **EP 2 559 763 B1**
(45) Date of publication and mention of the grant of the patent: **15.02.2017**
(21) Application number: 12176945.9
(22) Date of filing: 14.04.2005
(51) Int. Cl.: C12N 15/09, C12N 15/11

(54) **HLA-binding peptides, precursors thereof, DNA fragments and recombinant vectors that code for those peptide sequences**
HLA bindendes Peptid, dessen vorstufe, dafür codierendes DNA-Fragment und rekombinanter Vektor
Peptide de liaison HLA, précurseur, fragment d'ADN de codage identique et vecteur recombinant

(30) Priority: 30.04.2004 JP 2004135652; 17.09.2004 JP 2004272314; 25.02.2005 JP 2005050164
(43) Date of publication of application: 20.02.2013
(62) Divisional of application: 05730475.0
(73) Proprietor: NEC Corporation, Minato-ku, Tokyo 108-8001 (JP); Kochi University, Kochi 780-8520 (JP)
(72) Inventor: Miyakawa, Tomoya, Tokyo, 108-8001 (JP); Udaka, Keiko, Kochi, 783-8505 (JP)
(74) Representative: Vossius & Partner Patentanwälte Rechtsanwälte mbB

(56) References cited:
- WO-A1-99/45954
- WO-A1-2005/028503
- WO-A2-02/089731
- WO-A2-03/097677
- WO-A2-2004/111082
- WO-A2-2005/042698
- CN-A- 1 464 062
- KR-B1- 100 190 910
- US-B1- 6 709 828

## Description

### TECHNICAL FIELD

The present invention relates to an HLA-binding peptide, precursor thereof, a DNA segment and a recombinant vector coding for the HLA-binding peptide for use as a medicament.

### BACKGROUND ART

When one is infected with a virus such as hepatitis C virus (HCV) a virus specific immune response is induced to eliminate the virus following a defense by the innate immune system.

When a specific immune response is induced isolated viral particles lose their infectivity by neutralizing antibodies and are subsequently eliminated. In the other words, virus infected cells are lysed by cytotoxic T lymphocytes (CTLs). CTL recognizes as antigen an epitope peptide presented by an HLA class I molecule. Such epitope peptides are 8 to 11 amino acids in length. Therefore, it is critical to identify viral epitope peptides in order to develop a therapeutic vaccine against the virus.

A technique of this kind is known from Patent Publication 1. Patent Publication 1 states that an oligopeptide formed from a specific amino acid sequence has the property of binding to an HLA.
[Patent Publication 1] Japanese Patent Application Laid-open No. H8-151396 (1996)

### DISCLOSURE OF THE INVENTION

However, the conventional technique described in the above-mentioned publication has room for improvement on the following points.

Firstly, it is unclear whether or not the HLA-binding peptide of the above-mentioned publication binds to an HLA molecule effectively, and there is still room for improvement in terms of the property of binding to an HLA.

Secondly, it is stated that the HLA-binding peptide of the above-mentioned publication has the property of binding to HLA-DQ4. However, it is unclear whether or not it binds to an HLA-A2 type molecule (product of the HLA-A*0201 gene and the like), which is often seen in European and American people, and an HLA-A24 type molecule (product of the HLA-A*2402 gene and the like), which is often seen in Japanese people.

The present invention has been accomplished under the above-mentioned circumstances, and provides HLA-binding peptides that exhibit high-affinity binding to a specific type of HLA molecule.

In particular, the present invention provides:
provided an HLA-binding peptide binding to an HLA-A type
   1. An HLA-binding peptide consisting of the amino acid sequence SEQ ID NO: 17 for use as a medicament, wherein said HLA-binding peptide binds to a human HLA-A24 type molecule.
   2. A DNA segment comprising a DNA sequence coding for the HLA-binding peptide defined in item 1 for use as a medicament, wherein said HLA-binding peptide binds to a human HLA-A24 type molecule.
   3. A recombinant vector comprising a DNA sequence coding for the HLA-binding peptide defined in item 1 for use as a medicament, wherein said HLA-binding peptide binds to a human HLA-A24 type molecule.
   4. An HLA-binding peptide precursor changing into the HLA-binding peptide defined in item 1 within a mammalian body for use as a medicament, wherein said HLA-binding peptide binds to a human HLA-A24 type molecule.

In particular, the present invention provides:
1. An HLA-binding peptide consisting of the amino acid sequence SEQ ID NO: 17 for use as a medicament, wherein said HLA-binding peptide binds to a human HLA-A24 type molecule.
2. A DNA segment comprising a DNA sequence coding for the HLA-binding peptide defined in item 1 for use as a medicament, wherein said HLA-binding peptide binds to a human HLA-A24 type molecule.
3. A recombinant vector comprising a DNA sequence coding for the HLA-binding peptide defined in item 1 for use as a medicament, wherein said HLA-binding peptide binds to a human HLA-A24 type molecule.
4. An HLA-binding peptide precursor changing into the HLA-binding peptide defined in item 1 within a mammalian body for use as a medicament, wherein said HLA-binding peptide binds to a human HLA-A24 type molecule.

### BRIEF DESCRIPTION OF THE DRAWINGS

The above-mentioned object, other objects, features, and advantages will become more apparent from preferred embodiments explained below by reference to the attached drawing.

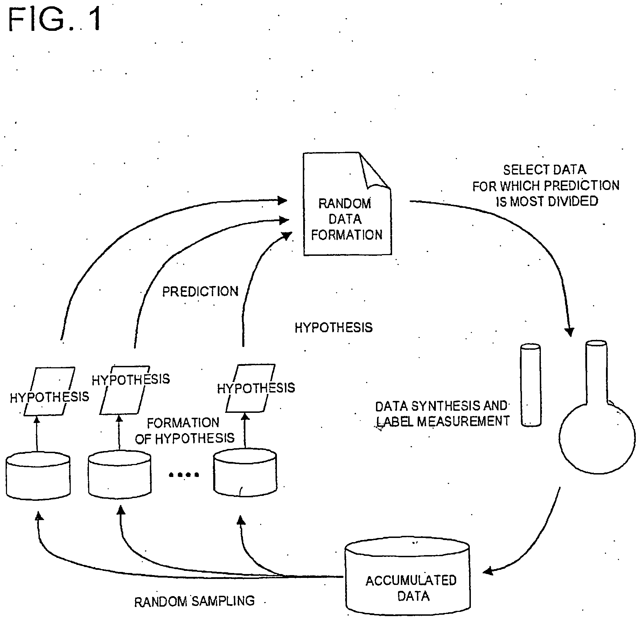

[FIG. 1] A schematic drawing for explaining an active learning experiment design used in an embodiment.

### BEST MODE FOR CARRYING OUT THE INVENTION

Modes for carrying out the present invention are explained below by reference to a drawing. In all the drawings, the same constitutional elements are denoted by the same reference numerals and symbols, so that the explanation will not be repeated.

### <Part 1>

In this part a peptide that contains an amino acid sequence for which the binding to an HLA molecule, predicted by a hypothesis obtained using an active learning experiment method (Japanese Patent Application Laid-open No. H11-316754 (1999)), is 3 or greater in terms of a -log Kd value, and consists of not less than 8 and not more than 11 amino acid residues is used as a candidate for an HLA-binding peptide. As a result of a binding experiment, it has been confirmed that these peptides are actually HLA-binding peptides.

As a result, a large number of HLA-binding peptides that have excellent properties in binding to an HLA-A type molecule because they contain an amino acid sequence for which the binding to the HLA molecule in terms of a-log Kd value is 3 or greater could be obtained efficiently.

The HLA-binding peptide described herein is an HLA-binding peptide that binds to an HLA-A type molecule, and that contains at least one type of amino acid sequence selected from the group consisting of SEQ ID NOS: 1 to 183, which will be described later, and that consists of not less than 8 and not more than 11 amino acid residues.

Among human HLA-A types, about 50% of Japanese people have the HLA-A24 type. For European and American people such as German people have the HLA-A2 type.

All of these sequences herein mentioned are sequences consisting of 9 amino acid residues contained in a certain genome protein of HCV (hepatitis C virus).

The sequences of SEQ ID NOS: 1 to 44 are given in Table 1 below.

**TABLE. 1**

| H L A → A 2 4 - BINDING PEPTIDE | | | | |
|---|---|---|---|---|
| SEQ ID NO | D90208 PREDICTED SCORE | PREDICTED SCORE | SEQ NAME | BINDING EXPERIMENT DATA |
| 1 | ILPGSFTTL | 6.9039 | 674 | 7.6571 |
| 2 | VILDSFDPI | 6, 293 | 2251 | 5.32417 |
| 3 | RYAPVCKPL | 6.2755 | 2132 | 6.14848 |
| 4 | FWAKHMWNF | 6.0822 | 1760 | |
| 5 | ALYDVVSTL | 6.0484 | 2593 | 6.38942 |
| 6 | TVLSDFKTW | 6.0021 | 1986 | |
| 7 | PYIEQGMQL | 5.9628 | 1716 | |
| 8 | WHYPCTVNF | 5.921 | 616 | 6.38729 |
| 9 | KFPPALPIW | 5.8662 | 2280 | |
| 10 | TYSTYCKFL | 5.8658 | 1292 | |
| 11 | AYSQQTRGL | 5.831 | 1031 | |
| 12 | AQPGYPWPL | 5.8258 | 77 | 5.36419 |
| 13 | ILMTHFFSI | 5.8071 | 2843 | 7.89519 |
| 14 | SYTWTGALI | 5.8059 | 2422 | 7.12954 |
| 15 | SPPAVPQIF | 5.7982 | 1215 | |
| 16 | LLPRRGPRL | 5.7503 | 36 | 7.71195 |
| 17 | ALYGVWPLL | 5.7447 | 789 | 6.98038 |
| 18 | LMTHFFSIL | 5.7443 | 2844 | 5.9169 |
| 19 | LLKRLHQWT | 5.7425. | 1956 | 6.857254 |
| 20 | YILLLFLLL | 5.738 | 718 | |
| 21 | ARPDYNPPL | 5.7226 | 2289 | |
| 22 | AYYSMVGNW | 5.7076 | 360 | 6.46991 |
| 23 | FLARLLWWL | 5.6847 | 838 | 6.17696 |
| 24 | SQLDLSGWF | 5.6728 | 2962 | |
| 25 | SMLTDPSHI | 5.6657 | 2173 | 6.94013 |
| 26 | EYILLLFLL | 5.6643 | 717 | |
| 27 | ILLGPADSF | 5.6526 | 1010 | 5.50208 |
| 28 | LNPSVAATL | 5.6281 | 1254 | |
| 29 | GLLSFLVFF | 5.6228 | 764 | |
| 30 | YVYDHLTPL | 5.6148 | 948 | |
| 31 | HYAPRPCGI | 5.5954 | 488 | |
| 32 | GLIHLHRNI | 5.595 | 688 | |
| 33 | HYRDVLKEM | 5.5928 | 2482 | 2482 |
| 34 | YYKVFLARL | 5.5825 | 834 | 7.24746 |
| 35 | CMVDYPYRL | 5.566 | 607 | |
| 36 | AVIPDREVL | 5.5541 | 1693 | |
| 37 | NPSRCWVAL | 5.5313 | 234 | 6.28275 |
| 38 | VFSDMETKL | 5.5347 | 975 | 7.30704 |
| 39 | VWPLLLLLL | 5.5297 | 793 | |
| 40 | ITYSTYCKF | 5.5171 | 1291 | 6.97507 |
| 41 | IEPLDLPQI | 5.5049 | 2873 | |
| 42 | LLSTTEWQI | 5.4989 | 666 | 8.33563 |
| 43 | PLLREEVVF | 5. 4208 | 2139 | |
| 44 | ATPPGSITY | 4.2466 | 1349 | |

The sequences of SEQ 10 NOS: 1 to 44 are sequences consisting of 9 amino acid residues contained in a certain genome protein (SEQ ID NO: 184) of the HCV D90208 strain, which will be described later. The sequences of SEQ ID NOS: 1 to 44 are sequences predicted by the above-mentioned method to have superior binding to an HLA-A24 type molecule. SEQ ID NOS: 1 to 44 are arranged in decreasing binding order. That is, SEQ ID NO: 1 is the sequence that is predicted to have the best binding. A predicted score for binding to the HLA-A24 type molecule and binding experiment data for each sequence are expressed in the form of -log Kd values.

The sequences of SEQ ID NOS: 45 to 83 are given in Table 2 below.

**TABLE. 2**

| H L A - A 2 4 - BINDING PEPTIDE | | | | |
|---|---|---|---|---|
| SEQ ID NO | D89815 PREDICTED SCORE | PREDICTED SCORE | SEQ NAME | BINDING EXPERIMENT DATA |
| 45 | VILDSFEPL | 6.4276 | 2251 | 5.00343 |
| 46 | ILPCSYTTL | 6.131 | 674 | |
| 47 | FWAKHMWNF | 6.0822 | 1760 | |
| 48 | ALYDVVSTL | 6.0484 | 2593 | 6.38942 |
| 49 | AFYGVWPLL | 5.9676 | 789 | 7.7344 |
| 50 | PYIEQGMQL | 5.9628 | 1716 | |
| 51 | TPPAVPQTF | 5.9302 | 1215 | |
| 52 | WHYPCTVNF | 5.921 | 616 | 6.38729 |
| 53 | GILPFFMFF | 5.9182 | 764 | 7.69551 |
| 54 | GLIHLHQNI | 5.879 | 688 | 5.85566 |
| 55 | LMCAVHPEL | 5.8442 | 876 | 6.59126 |
| 56 | TVLADFKTW | 5.8411 | 1986 | 6.51874 |
| 57 | AYSQQTRGL | 5.831 | 1031 | |
| 58 | AQPGYPWPL | 5.8258 | 77 | 5.36419 |
| 59 | PLLRDEVTF | 5.8128 | 2139 | 5.08926 |
| 60 | ILMTHFFSI | 5.8071 | 2843 | 7.89519 |
| 61 | SYTWTGALI | 5.8059 | 2422 | 7.12954 |
| 62 | ATPPGSVTF | 5.7779 | 1349 | 6.51124 |
| 63 | LLPRRGPRL | 5.7503 | 36 | 7.71195 |
| 64 | LMTHFFSIL | 5.7443 | 2844 | 5.9169 |
| 65 | LLKRLHQWI | 5.7425 | 1956 | 6.85724 |
| 66 | ARPDYNPPL | 5.7226 | 2289 | |
| 67 | AYYSMVGNW | 5.7076 | 360 | 6.46991 |
| 68 | KFPAAMPVW | 5.7062 | 2280 | |
| 69 | QYTLLFNIL | 5.7028 | 1804 | |
| 70 | LVPGAAYAF | 5.6865 | 782 | 782 |
| 71 | RYAPACKPL | 5.685 | 2132 | 6.75756 |
| 72 | PLARLIWWL | 5.6847 | 838 | 6.17696 |
| 73 | SQLDLSGWF | 5.6728 | 2962 | |
| 74 | SMLTDPSHI | 5.6657 | 2173 | 6.94014 |
| 75 | ILLGPADSF | 5.6526 | 1010 | 5.50208 |
| 76 | WLRDVWDWI | 5.6315 | 1976 | 6.34379 |
| 77 | YVVLLFLLL | 5.6308 | 718 | |
| 78 | LNPSVAATL | 5.6281 | 1254 | |
| 79 | YVYDHLTPL | 5.6148 | 948 | |
| 80 | HYRDVLKEM | 5.5928 | 2482 | |
| 81 | TLRRHVDLL | 5.5762 | 257 | |
| 82 | AVIPDREVL | 5.5541 | 1693 | |
| 83 | FLISQLFTF | 5.5528 | 285 | |

The sequences of.SEQ ID NOS: 45 to 83 are sequences consisting of 9 amino acid residues contained in a certain genome protein (SEQ ID NO: 1885) of the HCV D89815 strain. The sequences of SEQ ID NOS: 45 to 83 are sequences predicted by the above-mentioned method to have superior binding to an HLA-A24 type molecule. SEQ ID NOS: 45 to 83 are arranged in decreasing binding order. That is, SEQ ID NO: 45 is the sequence that is predicted to have the best binding. A predicted score for binding to the HLA-A24 type molecule and binding experiment data for each sequence are expressed in the form of -log Kd values.

The sequences of SEQ ID NOS: 84 to 123 are shown in Table 3 below.

**TABLE. 3**

| H L A - A 2 4 - BINDING PEPTIDE | | | | |
|---|---|---|---|---|
| SEQ ID NO | PREDICTED pBRT703' X SCORE | PREDICTED SCORE | SEQ NAME | BINDING EXPERIMENT DATA |
| 84 | VILDSFEPL | 8.7012 | 2251 | 5.00343 |
| 85 | ILPCSYTTL | 6.2441 | 74 | |
| 86 | GILPFFMFF | 6.1234 | 764 | 7.69551 |
| 87 | PLLRDEVTF | 6.0954 | 2139 | 5.08926 |
| 88 | TPPAVPQTF | 6.0934 | 1215 | |
| 89 | FWAKHKWMF | 6.0822 | 1760 | |
| 90 | TVLADFKTW | 5.9355 | 1986 | 6.51874 |
| 91 | ALYDVVSTL | 5.9179 | 2593 | 6.38942 |
| 92 | WEYPCTVNF | 5.8742 | 616 | 6.38728 |
| 93 | ATPPGSVTF | 5.8681 | 1349 | 6.51124 |
| 94 | GLIHLHQNI | 5.8476 | 688 | 5.85666 |
| 95 | AYSQQTRGL | 5.831 | 1031 | |
| 96 | ILMTHFFSI | 5.8217 | 2843 | 7.89519 |
| 97 | FLARLIWWL | 5.7815 | 838 | 6.17696 |
| 98 | AFYGVWPLL | 5.7373 | 789 | 7.7344 |
| 99 | FLISQLFTF | 5.7341 | 285 | |
| 100 | ILLGPADSF | 5.719 | | 5.50208 |
| 101 | SMLTDPSH1 | 5.6922 | 2173 | 6.94014 |
| 102 | AYYSMVGNW | 5.6746 | 360 | 6.46991 |
| 103 | QYTLLFNIL | 5.6682 | 1804 | |
| 104 | LLKRLHQWI | 6.6343 | 1966 | 6.85724 |
| 105 | SQLDLSGWF | 5.5993 | 2962 | |
| 106 | WLRDVWDWI | 5.5818 | 1976 | 5.34379 |
| 107 | ITYSTYGKF | 5.5352 | 1291 | 6.37373 |
| 108 | SYTWTGALI | 5.5253 | 2422 | 7.12954 |
| 109 | LLSTTEWQI | 5.5182 | 668 | 8.33563 |
| 110 | RYAPACKPL | 5.5076 | 2132 | 6.75756 |
| 111 | PLIWWLQYF | 5.5035 | 841 | |
| 112 | VLADFKTWL | 5.4871 | 1987 | 6.63423 |
| 113 | LVPGAAYAF | 5.4661 | 782 | |
| 114 | DLPQIIQRL | 5.4605 | 2877 | |
| 115 | WICTVLADF | 5.4521 | 1983 | |
| 116 | FYGVWPLLL | 5.4409 | 790 | |
| 117 | LLLSILGPL | 5.4365 | 891 | |
| 118 | HYRDVLKEM | 5.4331 | 2482 | |
| 119 | LIWWLQYFI | 5.4328 | S42 | |
| 120 | AVIPDREVL | 5.4247 | 1693 | |
| 121 | TRPPHGNWF | 5.4243 | 542 | |
| 122 | KFPAAMPVW | 5.424 | 2280 | |
| 123 | VFPDLGVRV | 5.3898 | 2580 | 6.73918 |

The sequences of SEQ ID NOS: 84 to 123 are sequences consisting of 9 amino acid residues contained in a certain genome protein (SEQ ID NO: 186) of the HCV pBRT703'X strain, which will be described later. The sequences of SEQ ID NOS: 84 to 123 are sequences predicted by the above-mentioned method to have superior binding to an HLA-A24 type molecule. SEQ ID NOS: 84 to 123 are arranged in decreasing binding order. That is, SEQ ID NO: 84 is the sequence that is predicted to have the best binding. A predicted score for the binding to the HLA-A24 type molecule and binding experiment data for each sequence are expressed in the form of -log Kd values.

The sequences of SEQ ID NOS: 124 to 183 are shown in Table 4 below.

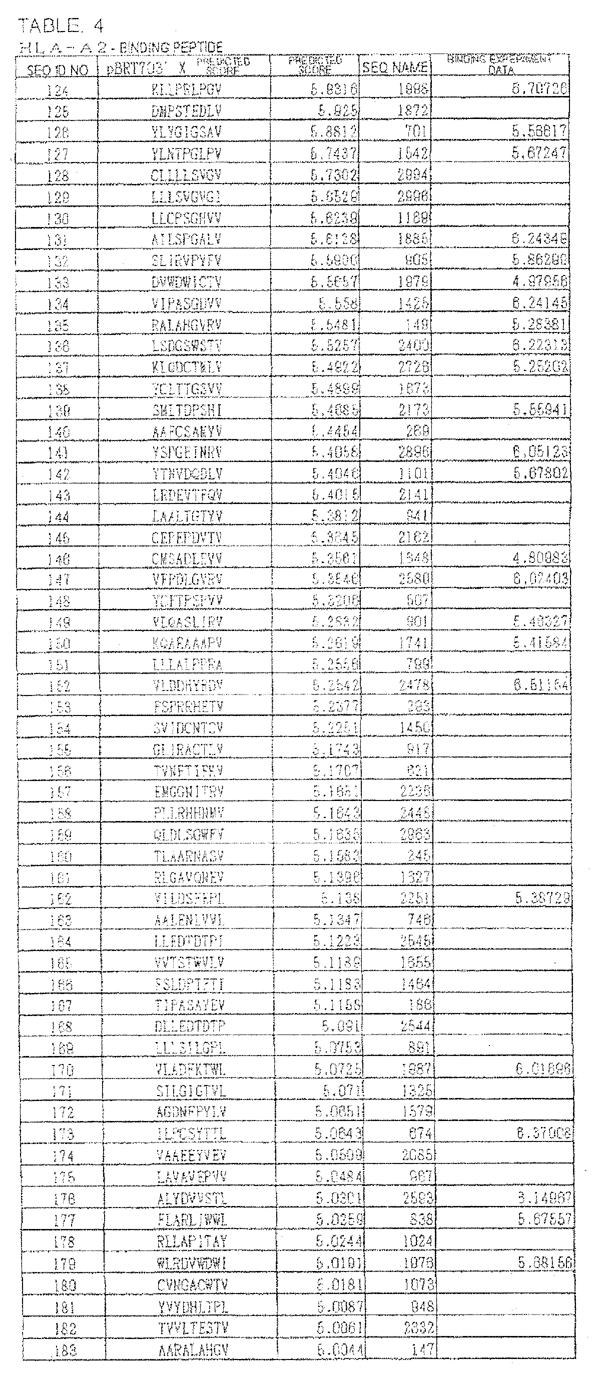

The sequences of SEQ ID NOS: 124 to 183 are sequences consisting of 9 amino acid residues contained in a certain genome protein (SEQ ID NO: 186) of the HCV pBRT703'X strain, which will be described later. The sequences of SEQ ID NOS: 124 to 183 are sequences predicted by the above-mentioned method to have superior binding to an HLA-A2 type molecule. SEQ ID NOS: 124 to 183 are arranged in decreasing binding order. That is, SEQ ID NO: 124 is the sequence that is predicted to have the best binding. A predicted score for the binding to the HLA-A2 type molecule and binding experiment data for each sequence are expressed in the form of -log Kd values.

Although details are described later, it is clear that in all of Table 1 to Table 4, there is a correlation between the predicted score and the binding experiment data. That is, although there are slight errors, it can be said that a peptide that is predicted by the above-mentioned method to have high binding to the HLA-A type molecule is found experimentally to have high binding to the HLA-A type molecule.

Since there is no conventional technique for discovering an HLA-binding peptide by utilizing such an experimental design method, there are only a very small number of HLA-binding peptides that have been experimentally confirmed to have HLA-binding properties. Because of this, even when a peptide consisting of 9 amino acid residues is randomly synthesized by a conventional method, and subjected to an experiment to find out if it binds to an HLA molecule, there is a probability of only about 1 in 100 of finding one that has a binding in terms of a -log Kd value, exceeding 6.

Since the technique of finding an HLA-binding peptide by utilizing the experimental design method is used, as described above, as many as 183 sequences of HLA-binding peptides can be found. Furthermore, when the binding of some of the HLA-binding peptides obtained is experimentally examined, it is confirmed that all of the sequences that have been subjected to the experiment exhibit an excellent binding to HLA that is equal to or higher than that predicted.

Among these sequences, an HLA-binding peptide containing at least one type of amino acid sequence selected from the group consisting of SEQ ID NOS: 1, 2, 3, 5, 8, 12, 13, 14, 16, 17, 18, 19, 22, 23, 25, 27, 34, 37, 38, 40, 42, 45, 48, 49, 52, 53, 54, 55, 56, 58, 59, 60, 61, 62, 63, 64, 65, 67, 71, 72, 74, 75, 76, 84, 86, 87, 90, 91, 92, 93, 94, 96, 97, 98, 100, 101, 102, 104, 106, 107, 108, 109, 110, 112, 123, 124, 126, 127, 131, 132, 133, 134, 135, 136, 137, 139, 141, 142, 146, 147, 149, 150, 152, 162, 170, 173, 176, 177, and 179 is experimentally confirmed to bind to a human HLA-A type molecule. It can therefore be said with certainty that it is an HLA-binding peptide that has excellent properties in binding to a human HLA-A type molecule.

The binding to an HLA molecule of the HLA-binding peptide described herein is 3 or greater in term of a -log Kd value, 5 or greater, or 5.4 or greater.

In the field of biochemistry, it is known that a binding ability in terms of a -log Kd value, of about 3 is the threshold level for whether or not a peptide actually binds to an MHC such as an HLA. Therefore, if the binding to an HLA molecule in terms of a -log Kd value, is 3 or greater, it can be said That it is an HLA-binding peptide.

Furthermore, if the binding to an HLA molecule in terms of a -log Kd value, is 5 or greater, since the peptide obtained has excellent properties in binding to the HLA molecule, it can suitably be used for development of an effective therapeutic drug and/or prophylactic drug for, e.g., an immune disease.

Moreover, if the binding to an HLA molecule in terms of a -log Kd value, is 5.4 or greater, the peptide obtained has particularly good properties in binding to the HLA molecule, and it can suitably be used for the development of an even more effective therapeutic drug and/or preventive drug for, e.g., an immune disease.

Furthermore, it may be arranged that the HLA-binding peptide described herein consists of not less than 8 and not more than 11 amino acid residues.

In this way, if the peptide consists of not less than 8 and not more than 11 amino acid residues, it has excellent properties in binding to an HLA molecule. Furthermore, the cytotoxic T lymphocyte (CTL) specifically recognizes a virus antigen (CTL epitope) consisting of 8 to 11 amino acids presented in an HLA class I molecule on the surface of a cell infected with a virus, and eliminates the virus by damaging the infected cell. It is important to prepare such a CTL epitope consisting of 8 to 11 amino acids that is specific to a virus in order to prepare a vaccine for therapy or prevention against the virus.

For example, the above-mentioned HLA-binding peptide may be a peptide consisting of amino acid residues alone.

For example, it may be an HLA-binding peptide precursor that is optionally modified with a sugar chain or a fatty acid group as long as the effects of the present disclosure are not impaired. Such a precursor is subjected to a change involving digestion by a digestive enzyme in a living mammalian body such as in a human digestive organ to become an HLA-binding peptide, thus exhibiting the similar effects to those shown by the above-mentioned HLA-binding peptide.

Furthermore, the above-mentioned HLA-binding peptide may be a peptide that binds to a human HLA-A24 type molecule.

Moreover, the above-mentioned HLA-binding peptide may be a peptide that binds to a human HLA-A2 type molecule.

In accordance with this constitution, since a peptide is obtained that binds to an HLA-A24 type molecule, which is often seen in Asian people, such as Japanese people, it can be utilized in the development of a therapeutic drug and/or a preventive drug that is particularly effective for Asian people, such as Japanese people.

Furthermore, in accordance with this constitution, since a peptide is obtained that binds to an HLA-A2 type molecule, which is often seen in European and American people in addition to Japanese people, it can be utilized in the development of a therapeutic drug and/or a preventive drug that is particularly effective for European and American people in addition to Japanese people.

The amino acid sequence contained in the above-mentioned HLA-binding peptide may be an amino acid sequence derived from a certain genome protein of HCV.

### <Part 2>

In accordance with this part, there is disclosed an HLA-binding peptide that binds to an HLA-A type molecule, contains an amino acid sequence formed by deletion, substitution, or addition of one or two amino acid residues of the amino acid sequence contained in the above-mentioned HLA-binding peptide, and consists of not less than 8 and not more than 11 amino acid residues.

As described later, even though the constitution includes an amino acid sequence formed by deletion, substitution, or addition of one or a few amino acid residues of a specific amino acid sequence that binds to an HLA-A type molecule, the similar effects to those of the HLA-binding peptide related to the above-mentioned part 1 are exhibited.

Although the amino acid sequences of polyproteins of the above-mentioned HCV D90208 strain, D89815 strain, and pBRT703'X strain (mutant subclone of D89815) are different from each other in part, since the correlation between predicted data and experimental data for the -log Kd value of several 9-mer peptides existing in a certain genome protein of the D90208 strain is high, that is, a sequence that is determined to be binding based on predicted data shows a good -log Kd value in experimental data, it can be predicted that the D89815 strain and the pBRT703'X strain (mutant subclone of D89815) will show a -log Kd value with a superior ranking in the predicted data. Therefore, it can be predicted that even amino acid sequences in a certain genome proteins of the D89815 strain and the pBRT703'X strain (mutant subclone of D89815), which are amino acid sequences formed by substitution of one or two amino acid residues of the amino acid sequences that exhibit binding properties, will similarly show excellent HLA-binding properties.

That is, it can be predicted that even an amino acid sequence formed by deletion, substitution, or addition of one or two amino acid residues of an amino acid sequence shown in SEQ ID NOS: 1 to 183 that has excellent properties in binding to an HLA-A type molecule will show excellent HLA-binding properties in the similar manner.

From another viewpoint, it can be predicted that even an amino acid sequence formed by deletion, substitution, or addition of one or a few amino acid residues of an amino acid sequence predicted by the above-mentioned method to have excellent properties in binding to an HLA-A type molecule will show excellent HLA-binding properties in the similar manner. The amino acid residues that are substituted may be amino acid residues having similar properties to each other, such that both are hydrophobic amino acid residues.

Moreover, the HLA-binding peptides described in parts 1 and 2 can be produced using a method known to a person skilled in the art. For example, they may be artificially synthesized by a solid-phase method or a liquid-phase method. Alternatively, these HLA-binding peptides may be produced by expressing them from a DNA segment or a recombinant vector coding for these HLA-binding peptides. These HLA-binding peptides thus obtained can be identified by a method known to a person skilled in the art. For example, identification is possible by use of Edman degradation and/or mass spectrometry.

### <Part 3>

In accordance with the present part, there is disclosed a DNA segment containing a DNA sequence coding for the above-mentioned HLA-binding peptide. Since the DNA segment related to the present part contains a specific DNA sequence, it can express the above-mentioned HLA-binding peptide.

When the above-mentioned HLA-binding peptide is expressed by using the DNA segment related to the present part, expression may be carried out by incorporating this DNA segment into a cell, or expression may be carried out by using a commercial artificial protein expression kit.

Furthermore, continuous expression may be carried out by incorporating the above-mentioned DNA segment into, for example, a human cell. Because of this, an HLA-binding peptide can be made to be present constitutively within a cell by incorporating a DNA segment coding for the HLA-binding peptide into the cell rather than incorporating the HLA-binding peptide itself into the cell. When an HLA-binding peptide is used as a vaccine, such an ability to express constitutively is advantageous in terms of enhancing the efficacy of the vaccine.

Moreover, the DNA segment related to the present part can be produced by a method known to a person skilled in the art. For example, it may be artificially synthesized by means of a commercial DNA synthesizer.

Alternatively, it may be segmented from the HCV genome by using a restriction enzyme. Alternatively, it may be amplified from the HCV genome by a PCR method using a pair of primers. The DNA segment thus obtained may be identified using a method known to a person skilled in the art. For example, it may be identified by a commercial DNA sequencer.

### <Part 4>

In accordance with the present part, there is disclosed a recombinant vector that contains a DNA sequence coding for the above-mentioned HLA-binding peptide. Since the recombinant vector related to the present part contains a specific DNA sequence, the above-mentioned HLA-binding peptide can be expressed.

When the above-mentioned HLA-binding peptide is expressed by using the recombinant vector related to the present part, expression may be carried out by incorporating this recombinant vector into a cell, or expression may be carried out by using a commercial. artificial protein expression kit.

Furthermore, continuous expression may be carried out by incorporating the above-mentioned recombinant vector into, for example, a human cell. Because of this, the HLA-binding peptide can be made to be present continuously within a cell by incorporating a recombinant vector coding for the HLA-binding peptide into the cell rather than incorporating the HLA-binding peptide itself into the cell. When the HLA-binding peptide is used as a vaccine, such an ability to express continuously is advantageous in terms of enhancing the efficacy of the vaccine.

Furthermore, in the above-mentioned recombinant vector, the amount of HLA-binding peptide expressed can be controlled with high precision by the use of a certain sequence in a regulatory region involved in transcription and expression, such as a promoter region upstream of a DNA sequence coding for the above-mentioned HLA-binding peptide. Moreover, the number of copies of a recombinant vector in a cell. can be controlled with high precision by the use of a certain sequence in a regulatory region involved in replication, such as the origin region of the recombinant vector.

Furthermore, the above-mentioned recombinant vector may freely contain a sequence other than the DNA sequence coding for the above-mentioned HLA-binding peptide. For example, it may contain a sequence of a marker gene such as a drug resistance gene.

Moreover, the recombinant vector related to the present part can be produced using a method known to a person skilled in the art. For example, it may be obtained by cleaving a multicloning site of a commercial vector such as pBR322 or pUC19 at a certain restriction enzyme site, and inserting the above-mentioned DNA segment into the site and carrying out ligation. Furthermore, the recombinant vector thus obtained can be identified using a method known to a person skilled in the art. For example, it can be confirmed by agarose gel electrophoresis whether or not the length of the DNA segment cleaved by a predetermined restriction enzyme coincides with the restriction map of a commercial vector such as pBR322 or pUC19 and, furthermore, it can be identified by a DNA sequencer whether or not the above-mentioned DNA sequence is contained in the DNA sequence cut out from the multicloning site.

If an amino sequence that is predicted by the above-mentioned method to have excellent HLA-binding properties is obtained, it can be expected that it will exhibit excellent HLA-binding properties in the similar way when confirmation is carried out experimentally. Because of this, these HLA-binding peptides can suitably be used mainly for the therapy or prevention of infectious diseases (e.g. influenza, SARS, HIV, HCV), and also for cancer immunotherapy, allergic diseases (e.g. pollen allergy (hay fever), rheumatism, atopy, asthma) and/or autoimmune diseases.

### (Examples)

The present invention is further explained below by reference to Examples.

Specifically, procedures of prediction, experiment, and evaluation in the present examples were carried out based on an active learning experiment design, and in general the following steps were repeated. A schematic drawing for the active learning experiment design employed here is shown in FIG. 1.
(1) A trial of a lower-order learning algorithm, which will be described later, was carried out once. That is, a plurality of hypotheses were generate by random sampling from accumulated data and, with regard to randomly expressed candidate query points (peptide), a point that showed the largest distribution of predicted values was selected as a query point to be subjected to an experiment.
(2) The peptide at the selected query point was prepared by a synthesis and purification method, which will be described later, and the actual binding ability was measured by an experiment, which will be described later, and added to accumulated data.

In the present example, as the lower-order learning algorithm, a supervised learning algorithm of a Hidden Markov Model was used, and 20 to 30 types of peptides were predicted and selected per experiment by starting with the initial data for 223 types of peptides; the above-mentioned procedure was repeated four times, and a total of 341 data points were obtained.

More specifically, in the active learning method of the present example, 20 to 30 types of peptides containing an amino acid sequence in which 9 of 20 types of amino acids were arranged were designed and synthesized per experiment. The strength of binding (binding ability) thereof to an HLA molecule was measured. The binding ability (Kd value in molar concentration) was obtained as an experimental result. When the binding ability was high, the peptide was selected as a candidate for an HLA-binding peptide that could be used as a material for a vaccine.

The results thus obtained were inputted into a learning system equipped with a learning machine employing the Hidden Markov Model as a mathematical algorithm, and rules were created. The learning machine sampled different results to prepare the rules. The rules expressed by the learning machine had different constitutions. The rules thus obtained and experimental data were stored as needed as accumulated data.

From among more than 20⁹ = 500 billion peptide sequences, candidates for a subsequent experiment were selected by the rules, and the above-mentioned process was repeated. In this stage, different rules were applied to experimental candidates, and the candidates for which predictions of the experimental results were divided were subjected to experiment. In this way, since the candidates for which predictions of the experimental results were divided were subjected to subsequent experiment, the final precision of the prediction was increased.

In this way, a plurality of learning machines carried out selective sampling in which samples that would give different predictions were selected as experimental candidates, information could be gained efficiently, and a hypothesis (rule) with high precision could be obtained. Repeating the above-mentioned process four times gave excellent results as in Examples described later. Repeating it seven times or more gave even better results.

In accordance with such an active learning method, the number of repetitions of the binding experiment for peptides consisting of 9 amino acid residues, which would otherwise have to be carried out for the 500 billion or more combinations of all the candidates for HLA-binding peptides, could be reduced. In the active learning method, a rule was formed by experiment, and the experiment was repeated for tens of sequence candidates that were predicted by applying the rule. Because of this, the number of experiments could be cut, and the time and cost of the initial screening could be greatly reduced.

Furthermore, the hit rate for prediction of the binding of a peptide to HLA by the rule obtained by the active learning method reached 70 to 80%, whereas the hit rate by other known techniques such as the anchor method was as low as about 30%.

### <Synthesis and purification of peptide>

A peptide was manually synthesized by the Merrifield solid-phase method using Fmoc amino acids. After deprotection, reverse phase HPLC purification was carried out using a C18 column to give a purity of 95% or higher. Identification of the peptide and confirmation of its purity were carried out using a MALDI-TOF mass spectrometer (Voyager DE RP, PerSeptive). Quantitative analysis of the peptide was carried out by a Micro BCA assay (Pierce Corp.) using BSA as a standard protein.

### <Experiment of binding peptide to HLA-A24 type molecule>

The ability of a peptide to bind to an HLA-A24 type molecule, which is a product of the HLA-A*2402 gene, was measured using C1R-A24 cells expressing the HLA-A24 type molecule (cells prepared by Professor Masafumi Takiguchi, Kumamoto University being supplied with permission by Assistant Professor Masaki Yasukawa, Ehime University).

C1R-A24 cells were first exposed to acidic conditions at a pH of 3.3 for 30 seconds, thus dissociating and removing a light chain β2m, which is associated with HLA class I molecules in common, and an endogenous peptide originally bound to the HLA-A*2402 molecule. After neutralization, purified β2m was added to C1R-A24 cells, and the obtained product was added to serial dilutions of a peptide, and incubated on ice for 4 hours. Staining was carried out using fluorescently labeled monoclonal antibody 17A12, which recognizes association (MHC-pep) of the three members, That is, HLA-A*2402 molecule, the peptide, and β2m, which had reassociated during the incubation.

Subsequently, the MHC-pep count per C1R-A24 cell (proportional to the strength of fluorescence of the above-mentioned fluorescent antibody) was quantitatively measured using an FACScan fluorescence-activated cell sorter (Becton Dickinson Biosciences). A binding dissociation constant Kd value between the HLA-A24 type molecule and the peptide was calculated from the average strength of fluorescence per cell by a published method (Udaka et al., Immunogenetics, 51, 816-828, 2000).

### <Experiment of binding peptide to HLA-A2 type molecule>

The ability of a peptide to bind to an HLA-A2 type molecule, which is a product of the HLA-A*0201 gene, was measured using strain JY cells expressing the HLA-A*0201.

JY cells were first exposed to acidic conditions at a pH of 3.8 for 30 seconds, thus dissociating and removing a light chain β2m and an endogenous peptide, which were noncovalently associated with the HLA-A*0201 molecule. After neutralization, a reassociation experiment was carried out.

The above-mentioned JY cells and the purified β2m were added to stepped serial dilutions of peptide for which the binding ability would be measured, and incubation was carried out on ice for 4 hours. HLA-A*0201 molecules that had reassociated up to this point were stained using the associating type specific fluorescently-labeled monoclonal antibody BB7.2.

Subsequently, the amount of fluorescence per a cell was measured using a flow cytometer and a dissociation constant Kd value in molar concentration was calculated by a published method (Udaka et al., Immunogenetics, 51, 816-828, 2000).

### <Evaluation results>

The prediction results and the experimental results shown in Tables 1 to 4 above were obtained.

The sequences of SEQ ID NOS: 1 to 44 in Table 1 are sequences consisting of 9 amino acid residues contained in the full-length sequence of a certain genome protein of the HCV D90208 strain registered in the GenBank. Furthermore, the sequences of SEQ ID NOS: 1 to 44 are sequences having superior binding to an HLA-A24 type molecule as predicted by a hypothesis obtained by the experimental design method explained in Embodiment 1. SEQ ID NOS: 1 to 44 are arranged in decreasing binding order. That is, SEQ ID NO: 1 is the sequence that is predicted to have the best binding. The full-length amino acid sequence of the certain genome protein of the HCV D90208 strain is shown in SEQ ID NO: 184

The sequences of SEQ ID NOS: 45 to 83 are sequences consisting of 9 amino acid residues contained in the full-length sequence of a certain genome protein of the HCV D89815 strain registered in the GenBank. Furthermore, the sequences of SEQ ID NOS: 45 to 83 are sequences having superior binding to an HLA-A24 type molecule as predicted by a hypothesis obtained by the experimental design method explained in Embodiment 1. SEQ ID NOS: 45 to 83 are arranged in decreasing binding order. That is, SEQ ID NO: 45 is the sequence that is predicted to have the best binding. The full-length amino acid sequence of the certain genome protein of the HCV D89815 strain is shown in SEQ ID NO: 185

The sequences of SEQ ID NOS: 84 to 123 are sequences consisting of 9 amino acid residues contained in a certain genome protein of the HCV pBRT703'X strain (mutant subclone of D89815), obtained from Professor Yoshiharu Matsuura, at the research institute for Microbial diseases at Osaka University. Furthermore, the sequences of SEQ ID NOS: 84 to 123 are sequences having superior binding to an HLA-A24 type molecule as predicted by a hypotheses obtained by the experimental design method explained in Embodiment 1. SEQ ID NOS: 84 to 123 are arranged in decreasing binding order. That is, SEQ ID NO: 84 is the sequence that is predicted to have the best binding. The full-length amino acid sequence of the certain genome protein of the HCV pBRT703'X strain (mutant subclone of D89815) is shown in SEQ ID NO: 186

The sequences of SEQ ID NOS: 124 to 183 are sequences consisting of 9 amino acid residues contained in a certain genome protein of the above-mentioned HCV pBRT703'X strain (mutant subclone of D89815). Furthermore, the sequences of SEQ ID NOS: 124 to 183 are sequences having superior binding to an HLA-A2 type molecule as predicted by a hypotheses obtained by the experimental design method explained in Embodiment 1. SEQ ID NOS: 124 to 183 are arranged in decreasing binding order. That is, SEQ ID NO: 124 is the sequence that is predicted to have the best binding.

Table 1 to Table 4 show amino acid sequences that had superior scores in the predicted results obtained using the above-mentioned prediction program, the predicted score, and the corresponding binding experiment data for the HCV D90208 strain, the D89815 strain, and the pBRT703'X strain (mutant subclone of D89815). All of the binding experiment data were obtained by artificially synthesizing 9 amino acid peptides by the above-mentioned synthetic method.

Said certain genome proteins of the HCV D90208 strain and the D89815 strain are registered in the GenBank, but the sequences consisting of 9 amino acid residues therein, which are the HLA-binding peptides, are not currently registered.

Furthermore, the pDRT703'X strain (mutant subclone of D89815) is a mutant strain that is similar to a substrain of HCV often seen in Japanese hepatitis C patients. In the present example, an HLA-binding peptide contained in a certain genome protein of the mutant strain similar to the substrain often seen in Japanese people has been found. This HLA-binding peptide can suitably be used for the development of a hepatitis C therapeutic drug for Japanese people.

Here, the amino acid sequences of the certain genome proteins of the above-mentioned HCV D90208 strain, D89815 strain, and pBRT703'X strain (mutant subclone of D89815) are different from each other in part, and it can be predicted that even an amino acid sequence formed by substitution of one or a few amino acid residues in the amino acid sequence will similarly show excellent HLA-binding properties as described above.

For example, the sixth peptide from the left of SEQ ID NO: 1 of the D90208 strain is F, but it is Y for the peptide of SEQ ID NO: 46 of the D89815 strain and the peptide of SEQ ID NO: 85 of the pBRT703'X strain (mutant subclone of D89815).

Furthermore, the second peptide from the left of SEQ ID NO: 17 of the D90208 strain is L, but it is F for the peptide of SEQ ID NO: 49 of the D89815 strain and the peptide of SEQ ID NO: 98 of the pBRT703'X strain (mutant subclone of D89815).

Moreover, the fifth peptide from the left of SEQ ID NO: 3 of the D90208 strain is V, but it is A for the peptide of SEQ ID NO: 71 of the D89815 strain and the peptide of SEQ ID NO: 110 of the pBRT703'X strain (mutant subclone of D89815).

Furthermore, the seventh peptide from the left of SEQ ID NO: 2 of the D90208 strain is D, but it is E for the peptide of SEQ ID NO: 45 of the D89815 strain and the peptide of SEQ ID NO: 84 of the pBRT703'X strain (mutant subclone of D89315).

Moreover, the seventh peptide from the left of SEQ ID NO: 40 of the D90208 strain is C, but it is G for the peptide of SEQ ID NO: 107 of the pBRT703'X strain (mutant subclone of D89815).

Furthermore, the fifth peptide from the left of SEQ ID NO: 43 of the D90208 strain is E, but it is D for the peptide of SEQ ID NO: 59 of the D89815 strain and the peptide of SEQ ID NO: 87 of the pBRT703'X strain (mutant subclone of D89815), and the eighth peptide from the left of SEQ ID NO: 43 of the D90208 strain is V, but it is T for the peptide of SEQ ID NO: 59 of the D89815 strain and the peptide of SEQ ID NO: 87 of the pBRT703'X strain (mutant subclone of D89815).

Moreover, the seventh peptide from the left of SEQ ID NO: 44 of the D90208 strain is I, but it is V for the peptide of SEQ ID NO: 62 of the D89815 strain and the peptide of SEQ ID NO: 93 of the pBRT703'X strain (mutant subclone of D89815), and the ninth peptide from the left of SEQ ID NO: 44 of the D90208 strain is V, but it is F for the peptide of SEQ ID NO: 62 of the D89815 strain and the peptide of SEQ ID NO: 93 of the pBRT703'X strain.

Among the peptide sequences formed by substitution of one or two amino acid residues with each other, for example, the second peptide from the left of SEQ ID NO: 17 of the D90208 strain is L, but it is F for the peptide of SEQ ID NO: 49 of the D89815 strain and the peptide of SEQ ID NO: 98 of the pBRT703'X strain (mutant subclone of D89815), and the binding experimental value for the peptide of SEQ ID NO: 17 of the D90208 strain is 6.98038 whereas it is 7.7344 for the peptide of SEQ ID NO: 49 of the D89815 strain and the peptide of SEQ ID NO: 98 of the pBRT703'X strain (mutant subclone of D89815), thus confirming that they all show good binding properties.

Furthermore, among the peptide sequences formed by substitution of one or two amino acid residues with each other, for example, the seventh peptide from the left of SEQ ID NO: 40 of the D90208 strain is C, but it is G for the peptide of SEQ ID NO: 107 of the pBRT703'X strain (mutant subclone of D89815), and the binding experimental value for the peptide of SEQ ID NO: 40 of the D90208 strain is 6.97507 whereas it is 6.37373 for the peptide of SEQ ID NO: 107 of the pBRT703'X strain (mutant subclone of D89815), thus confirming that they all show good binding properties.

Moreover, among the peptide sequences formed by substitution of one or two amino acid residues with each other, for example, the fifth peptide from the left of SEQ ID NO: 3 of the D90208 strain is V, but it is A for the peptide of SEQ ID NO: 71 of the D89815 strain and the peptide of SEQ ID NO: 110 of the pBRT703'X strain (mutant subclone of D89815), and the binding experimental value for the peptide of SEQ ID NO: 3 of the D90208 strain is 6.14848 whereas it is 6.75756 for the peptide of SEQ ID NO: 71 of the D89815 strain and the peptide of SEQ ID NO: 110 of the pBRT703'X strain (mutant subclone of D89815), thus confirming that they all show good binding properties.

Furthermore, among the peptide sequences formed by substitution of one or two amino acid residues with each other, for example, the seventh peptide from the left of SEQ ID NO: 2 of the D90208 strain is D, but it is E for the peptide of SEQ ID NO: 45 of the D89815 strain and the peptide of SEQ ID NO: 84 of the pBRT703'X strain (mutant subclone of D89815), and the binding experimental value for the peptide of SEQ ID NO: 2 of the D90208 strain is 5.32417 whereas it is 5.00343 for the peptide of SEQ ID NO: 45 of the D89815 strain and the peptide of SEQ ID NO: 84 of the pBRT703'X strain (mutant subclone of D89815), thus confirming that they all show good binding properties.

Moreover, among the peptide sequences formed by substitution of one or two amino acid residues with each other, for example, the amino acid sequence is offset sideways by one between the peptide of SEQ ID NO: 90 and the peptide of SEQ ID NO: 112 of the pBRT703'X strain (mutant subclone of D89815), and the binding experimental value for the peptide of SEQ ID NO: 90 is 6.51874 whereas the binding experimental value for the peptide of SEQ ID NO: 112 is 6.63423, thus confirming that they all show good binding properties.

Furthermore, among the peptide sequences formed by substitution of one or two amino acid residues with each other, for example, the amino acid sequence is offset sideways by one between the peptide of SEQ ID NO: 13 of the D90208 strain and the peptide of SEQ ID NO: 60 of the D89815 strain and the peptide of SEQ ID NO: 18 of the D90208 strain and the peptide of SEQ ID NO: 64 of the D89815 strain, and the binding experimental value for the peptides of SEQ ID NOS: 13 and 60 is 7.89519 whereas the binding experimental value for the peptides of SEQ ID NOS: 18 and 64 is 5.9169, thus confirming that they all show good binding properties.

It can therefore be predicted that both the peptide sequences formed by substitution of one or two amino acid residues with each other will show excellent binding to an HLA-A24 type molecule. In conclusion, even an amino acid sequence formed by deletion, substitution, or addition of one or a few amino acid residues of an amino acid sequence that has excellent properties in binding to an HLA-A type molecule shown by SEQ ID NOS: 1 to 183 can be predicted to similarly show excellent HLA-binding properties.

From another viewpoint, even an amino acid sequence formed by deletion, substitution, or addition of one or a few amino acid residues of an amino acid sequence that has excellent properties in binding to an HLA-A type molecule as predicted by the hypothesis obtained by the experimental design method explained in Embodiment 1 similarly can be said to show excellent HLA-binding properties. The amino acid residues that are substituted are preferably amino acid residues that have similar properties to each other, such as the two being hydrophobic amino acid residues.

The present invention is explained above by reference to Examples.

These Examples are only illustrated as examples, and a person skilled in the art will understand that various modification examples are possible, and such modification examples are included in the scope of the present invention.

For example, in the above-mentioned Examples, HCV D90208 strain, D89815 strain, and pBRT703'X strain (mutant subclone of D80815) were used, but another HCV strain may be used. In this case, in accordance with the prediction program employed in the present invention, the HLA binding can be predicted with high precision.

Furthermore, the present disclosure describes the following:
1. An HLA-binding peptide binding to an HLA-A type molecule, said HLA-binding peptide comprising
   at least one type of amino acid sequence selected from the group consisting of SEQ ID NOS: 1 to 183, and
   not less than 8 and not more than 11 amino acid residues.
2. The HLA-binding peptide as set forth in item 1, comprising at least one type of amino acid sequence selected from the group consisting of SEQ ID NOS: 1, 2, 3, 5, 8, 12, 13, 14, 16, 17, 18, 19, 22, 23, 25, 27, 34, 37, 38, 40, 42, 45, 48, 49, 52, 53, 54, 55, 56, 58, 59, 60, 61, 62, 63, 64, 65, 67, 71, 72, 74, 75, 76, 84, 86, 87, 90, 91, 92, 93, 94, 96, 97, 98, 100, 101, 102, 104, 106, 107, 108, 109, 110, 112, 123, 124, 126, 127, 131, 132, 133, 134, 135, 136, 137, 139, 141, 142, 146, 147, 149, 150, 152, 162, 170, 173, 176, 177, and 179.
3. An HLA-binding peptide binding to an HLA-A type molecule, said HLA-binding peptide comprising
   an amino acid sequence formed by deletion, substitution, or addition of one or two amino acid residues of said amino acid sequence contained in the HLA-binding peptide as set forth in item 1 or 2, and
   not less than 8 and not more than 11 amino acid residues.
4. The HLA-binding peptide as set forth in any one of items 1 7 to 3, wherein said human HLA-binding peptide binds to a human HLA-A24 type molecule.
5. The HLA-binding peptide as set forth in any one of items 1 to 3, wherein said human HLA-binding peptide binds to a human HLA-A2 type molecule.
6. A DNA segment comprising a DNA sequence coding for the HLA-binding peptide as set forth in any one of items 1 to 5.
7. A recombinant vector comprising a DNA sequence coding for the HLA-binding peptide as set forth in any one of items 1 to 5.
8. An HLA-binding peptide precursor changing into the HLA-binding peptide as set forth in any one of items 1 to 5 within a mammalian body.

## Claims

1. An HLA-binding peptide consisting of the amino acid sequence SEQ ID NO: 17 for use as a medicament, wherein said HLA-binding peptide binds to a human HLA-A24 type molecule.

2. A DNA segment comprising a DNA sequence coding for the HLA-binding peptide defined in claim 1 for use as a medicament, wherein said HLA-binding peptide binds to a human HLA-A24 type molecule.

3. A recombinant vector comprising a DNA sequence coding for the HLA-binding peptide defined in claim 1 for use as a medicament, wherein said HLA-binding peptide binds to a human HLA-A24 type molecule.

4. An HLA-binding peptide precursor changing into the HLA-binding peptide defined in claim 1 within a mammalian body for use as a medicament, wherein said HLA-binding peptide binds to a human HLA-A24 type molecule.

## Patentansprüche

1. HLA bindendes Peptid, das aus der Aminosäuresequenz SEQ ID NO:17 besteht, zur Verwendung als Medikament, wobei das HLA bindende Peptid an ein humanes Molekül vom Typ HLA-A24 bindet.

2. DNA-Segment, das eine DNA-Sequenz umfasst, die das in Anspruch 1 definierte HLA bindende Peptid codiert, zur Verwendung als Medikament, wobei das HLA bindende Peptid an ein humanes Molekül vom Typ HLA-A24 bindet.

3. Rekombinanter Vektor, der eine DNA-Sequenz umfasst, die das in Anspruch 1 definierte HLA bindende Peptid codiert, zur Verwendung als Medikament, wobei das HLA bindende Peptid an ein humanes Molekül vom Typ HLA-A24 bindet.

4. HLA bindender Peptid-Vorläufer, der sich innerhalb eines Säugerkörpers in das in Anspruch 1 definierte HLA bindende Peptid umwandelt, zur Verwendung als Medikament, wobei das HLA bindende Peptid an ein humanes Molekül vom Typ HLA-A24 bindet.

## Revendications

1. Peptide de liaison HLA consistant en la séquence d'acides aminés SEQ ID NO: 17 pour utilisation en tant que médicament, lequel peptide de liaison HLA se lie à une molécule de type HLA-A24 humaine.

2. Segment d'ADN comprenant une séquence d'ADN codant le peptide de liaison HLA défini dans la revendication 1 pour utilisation en tant que médicament, dans lequel ledit peptide de liaison HLA se lie à une molécule de type HLA-A24 humaine.

3. Vecteur recombinant comprenant une séquence d'ADN codant le peptide de liaison HLA défini dans la revendication 1 pour utilisation en tant que médicament, dans lequel ledit peptide de liaison HLA se lie à une molécule de type HLA-A24 humaine.

4. Précurseur de peptide de liaison HLA se changeant en le peptide de liaison HLA défini dans la revendication 1 à l'intérieur d'un corps mammalien pour utilisation en tant que médicament, dans lequel ledit peptide de liaison HLA se lie à une molécule de type HLA-A24 humaine.


### SEQUENCE LISTING

<110> NEC Corporation. Kochi University
<120> HLA binding peptides
<130> 64004160
<160> 186
<170> PatentIn version 3.1
<210> 1
   <211> 9
   <212> PRT
   <213> Hepatitis C virus
<400> 1
   Ile Leu Pro Cys Ser Phe Thr Thr Leu
   1               5
<210> 2
   <211> 9
   <212> PRT
   <213> Hepatitis C virus
<400> 2
   Val Ile Leu Asp Ser Phe Asp Pro Ile
   1 5
<210> 3
   <211> 9
   <212> PRT
   <213> Hepatitis C virus.
<400> 3
   Arg Tyr Ala Pro Val Cys Lys Pro Leu
   1 5
<210> 4
   <211> 9
   <212> PRT
   <213> Hepatitis C virus
<400> .4
<210> 5
   <211> 9
   <212> PRT
   <213> Hepatitis C virus
<400> 5
   Ala Leu Tyr Asp Val Val Ser Thr Leu
   1 5
<210> 6
   <211> 9
   <212> PRT
   <213> Hepatitis C virus
<400> 6
   Thr Val Leu Ser Asp Phe Lys Thr Trp
   1 5
<210> 7
   <211> 9
   <212> PRT
   <213> Hepatitis C virus
<400> 7
   Pro Tyr Ile Glu Gln Gly Met Gln Leu
   1 5
<210> 8
   <211> 9
   <212> PRT
   <213> Hepatitis C virus
<400> 8
   Trp His Tyr Pro Cys Thr Val Asn Phe
   1 5
<210> 9
   <211> 9
   <212> PRT
   <213> Hepatitis C virus
<400> 9
   Lys Phe Pro Pro Ala Leu Pro Ile Trp
   1 5
<210> 10
   <211> 9
   <212> PRT
   <213> Hepatitis C virus
<400> 10
   Thr Tyr Ser Thr Tyr Cys Lys Phe Leu
   1 5
<210> 11
   <211> 9
   <212> PRT
   <213> Hepatitis C virus
<400> 11
   Ala Tyr Ser Gln Gln Thr Arg Gly Leu
   1 5
<210> 12
   <211> 9
   <212> PRT
   <213> Hepatitis C virus
<400> 12
   Ala Gln Pro Gly Tyr Pro Trp Pro Leu
   1 5
<210> 13
   <211> 9
   <212> PRT
   <213> Hepatitis C virus
<400> 13
   Ile Leu Met Thr His Phe Phe Ser Ile
   1 5
<210> 14
   <211> 9
   <212> PRT
   <213> Hepatitis C virus
<400> 14
   Ser Tyr Thr Trp Thr Gly Ala Leu Ile
   1 5
<210> 15
   <211> 9
   <212> PRT
   <213> Hepatitis C virus
<400> 15.
   Ser Pro Pro Ala Val Pro Gln Thr Phe
   1 5
<210> 16
   <211> 9
   <212> PRT
   <213> Hepatitis C virus
<400> 16
   Leu Leu Pro Arg Arg Gly Pro Arg Leu
   1 5
<210> 17
   <211> 9
   <212> PRT
   <213> Hepatitis C virus
<400> 17
   Ala Leu Tyr Gly Val Trp Pro Leu Leu
   1               5
<210> 18
   <211> 9
   <212> PRT
   <213> Hepatitis C virus
<400> 18
   Leu Met Thr His Phe Phe Ser Ile Leu
   1               5
<210> 19
   <211> 9
   <212> PRT
   <213> Hepatitis C virus
<400> 19
   Leu Leu Lys Arg Leu His Gln Trp Ile
   1               5
<210> 20
   <211> 9
   <212> PRT
   <213> Hepatitis C virus
<400> 20
   Tyr Ile Leu Leu Leu Phe Leu Leu Leu
   1               5
<210> 21
   <211> 9
   <212> PRT
   <213> Hepatitis C virus
<400> 21
   Ala Arg Pro Asp Tyr Asn Pro Pro Leu
   1               5
<210> 22
   <211> 9
   <212> PRT
   <213> Hepatitis C virus
<400> 22
   Ala Tyr Tyr Ser Met Val Gly Asn Trp
   1               5
<210> 23
   <211> 9
   <212> PRT
   <213> Hepatitis C virus
<400> 23
   Phe Leu Ala Arg Leu Ile Trp Trp Leu
   1 5
<210> 24
   <211> 9
   <212> PRT
   <213> Hepatitis C virus
<400> 24
   Ser Gln Leu Asp Leu Ser Gly Trp Phe
   1 5
<210> 25
   <211> 9
   <212> PRT
   <213> Hepatitis C virus
<400> 25
   Ser Met Leu Thr Asp Pro Ser His Ile
   1 5
<210> 26
   <211> 9
   <212> PRT
   <213> Hepatitis C virus
<400> 26
   Glu Tyr Ile Leu Leu Leu Phe Leu Leu
   1 5
<210> 27
   <211> 9
   <212> PRT
   <213> Hepatitis C virus
<400> 27
   Ile Leu Leu Gly Pro Ala Asp Ser Phe
   1 5
<210> 28
   <211> 9
   <212> PRT
   <213> Hepatitis C virus
<400> 28
   Leu Asn Pro Ser Val Ala Ala Thr Leu
   1 5
<210> 29
   <211> 9
   <212> PRT
   <213> Hepatitis C virus
<400> 29
   Gly Leu Leu Ser Phe Leu Val Phe Phe
   1 5
<210> 30
   <211> 9
   <212> PRT
   <213> Hepatitis C virus
<400> 30
   Tyr Val Tyr Asp His Leu Thr Pro Leu
   1 5
<210> 31
   <211> 9
   <212> PRT
   <213> Hepatitis C virus
<400> 31
   His Tyr Ala Pro Arg Pro Cys Gly Ile
   1 5
<210> 32
   <211> 9
   <212> PRT
   <213> Hepatitis C virus
<400> 32
   Gly Leu Ile His Leu His Arg Asn Ile
   1 5
<210> 33
   <211> 9
   <212> PRT
   <213> Hepatitis C virus
<400> 33
   His Tyr Arg Asp Val Leu Lys Glu Met
   1 5
<210> 34
   <211> 9
   <212> PRT
   <213> Hepatitis C virus
<400> 34
   Tyr Tyr Lys Val Phe Leu Ala Arg Leu
   1 5
<210> 35
   <211> 9
   <212> PRT
   <213> Hepatitis C virus
<400> 35
   Cys Met Val Asp Tyr Pro Tyr Arg Leu
   1 5
<210> 36
   <211> 9
   <212> PRT
   <213> Hepatitis C virus
<400> 36
   Ala Val Ile Pro Asp Arg Glu Val Leu
   1 5
<210> 37
   <211> 9
   <212> PRT
   <213> Hepatitis C virus
<400> 37
   Asn Phe Ser Arg Cys Trp Val Ala Leu
   1 5
<210> 38
   <211> 9
   <212> PRT
   <213> Hepatitis C virus
<400> 38
   Val Phe Ser Asp Met Glu Thr Lys Leu
   1 5
<210> 39
   <211> 9
   <212> PRT
   <213> Hepatitis C virus
<400> 39
   Val Trp Pro Leu Leu Leu Leu Leu Leu
   1 5
<210> 40
   <211> 9
   <212> PRT
   <213> Hepatitis C virus
<400> 40
   Ile Thr Tyr Ser Thr Tyr Cys Lys Phe
   1 5
<210> 41
   <211> 9
   <212> PRT
   <213> Hepatitis C virus
<400> 41
   Ile Glu Pro Leu Asp Leu Pro Gln Ile
   1 5
<210> 42
   <211> 9
   <212> PRT
   <213> Hepatitis C virus
<400> 42
   Leu Leu Ser Thr Thr Glu Trp Gln Ile
   1 5
<210> 43
   <211> 9
   <212> PRT
   <213> Hepatitis C virus
<400> 43
   Pro Leu Leu Arg Glu Glu Val Val Phe
   1 5
<210> 44
   <211> 9
   <212> PRT
   <213> Hepatitis C virus
<400> 44
   Ala Thr Pro Pro Gly Ser Ile Thr Val
   1 5
<210> 45
   <211> 9
   <212> PRT
   <213> Hepatitis C virus
<400> 45
   Val Ile Leu Asp Ser Phe Glu Pro Leu
   1 5
<210> 46
   <211> 9
   <212> PRT
   <213> Hepatitis C virus
<400> 46
   Ile Leu Pro Cys Ser Tyr Thr Thr Leu
   1 5
<210> 47
   <211> 9
   <212> PRT
   <213> Hepatitis C virus
<400> 47
   Phe Trp Ala Lys His Met Trp Asn Phe
   1 5
<210> 48
   <211> 9
   <212> PRT
   <213> Hepatitis C. virus
<400> 48
   Ala Leu Tyr Asp Val Val Ser Thr Leu
   1 5
<210> 49
   <211> 9
   <212> PRT
   <213> Hepatitis C virus
<400> 49
   Ala Phe Tyr Gly Val Trp Pro Leu Leu
   1 5
<210> 50
   <211> 9
   <212> PRT
   <213> Hepatitis C virus
<400> 50
   Pro Tyr Ile Glu Gln Gly Met Gln Leu
   1 5
<210> 51
   <211> 9
   <212> PRT
   <213> Hepatitis C virus
<400> 51
   Thr Pro Pro Ala Val Pro Gln Thr Phe
   1 5
<210> 52
   <211> 9
   <212> PRT
   <213> Hepatitis C virus
<400> 52
   Trp His Tyr Pro Cys Thr Val Asn Phe
   1 5
<210> 53
   <211> 9
   <212> PRT
   <213> Hepatitis C virus
<400> 53
   Gly Ile Leu Pro Phe Phe Met Phe Phe
   1 5
<210> 54
   <211> 9
   <212> PRT
   <213> Hepatitis C virus
<400> 54
   Gly Leu Ile His Leu His Gln Asn Ile
   1 5
<210> 55
   <211> 9
   <212> .PRT
   <213> Hepatitis C virus
<400> 55
   Leu Met Cys Ala Val His Pro Glu Leu
   1 5
<210> 56
   <211> 9
   <212> PRT
   <213> Hepatitis C virus
<400> 56
   Thr Val Leu Ala Asp Phe Lys Thr Trp
   1 5
<210> 57
   <211> 9
   <212> PRT
   <213> Hepatitis C virus
<400> 57
   Ala Tyr Ser Gln Gln Thr Arg Gly Leu
   1 5
<210> 58
   <211> 9
   <212> PRT
   <213> Hepatitis C virus
<400> 58
   Ala Gln Pro Gly Tyr Pro Trp Pro Leu
   1 5
<210> 59
   <211> 9
   <212> PRT
   <213> Hepatitis C virus
<400> 59
   Pro Leu Leu Arg Asp Glu Val Thr Phe
   1 5
<210> 60
   <211> 9
   <212> PRT
   <213> Hepatitis C virus
<400> 60
   Ile Leu Met Thr His Phe Phe Ser Ile
   1 5
<210> 61
   <211> 9
   <212> PRT
   <213> Hepatitis C virus
<400> 61
   Ser Tyr Thr Trp Thr Gly Ala Leu Ile
   1 5
<210> 62
   <211> 9
   <212> PRT
   <213> Hepatitis C virus
<400> 62
   Ala Thr Pro Pro Gly Ser Val Thr Phe
   1 5
<210> 63
   <211> 9
   <212> PRT
   <213> Hepatitis C virus
<400> 63
   Leu Leu Pro Arg Arg Gly Pro Arg Leu
   1 5
<210> 64
   <211> 9
   <212> PRT
   <213> Hepatitis C virus
<400> 64
   Leu Met Thr His Phe Phe Ser Ile Leu
   1 5
<210> 65
   <211> 9
   <212> PRT
   <213> Hepatitis C virus
<400> 65
   Leu Leu Lys Arg Leu His Gln Trp Ile
   1 5
<210> 66
   <211> 9
   <212> PRT
   <213> Hepatitis C virus
<400> 66
   Ala Arg Pro Asp Tyr Asn Pro Pro Leu
   1 5
<210> 67
   <211> 9
   <212> PRT
   <213> Hepatitis C virus
<400> 67
   Ala Tyr Tyr Ser Met Val Gly Asn Trp
   1 5
<210> 68
   <211> 9
   <212> PRT
   <213> Hepatitis C virus
<400> 68
   Lys Phe Pro Ala Ala Met Pro Val Trp
   1 5
<210> 69
   <211> 9
   <212> PRT
   <213> Hepatitis C virus
<400> 69
   Gln Tyr Thr Leu Leu Phe Asn Ile Leu
   1 5
<210> 70
   <211> 9
   <212> PRT
   <213> Hepatitis C virus
<400> 70
   Leu Val Pro Gly Ala Ala Tyr Ala Phe
   1 5
<210> 71
   <211> 9
   <212> PRT
   <213> Hepatitis C virus
<400> 71
   Arg Tyr Ala Pro Ala Cys Lys Pro Leu
   1 5
<210> 72
   <211>. 9
   <212> PRT
   <213> Hepatitis C virus
<400> 72
<210> 73
   <211> 9
   <212> PRT
   <213> Hepatitis C virus
<400> 73
   Ser Gln Leu Asp Leu Ser Gly Trp Phe
   1 5
<210> 74
   <211> 9
   <212> PRT
   <213> Hepatitis C virus
<400> 74
   Ser Met Leu Thr Asp Pro Ser His Ile
   1 5
<210> 75
   <211> 9
   <212> PRT
   <213> Hepatitis C virus
<400> 75
   Ile Leu Leu Gly Pro Ala Asp Ser Phe
   1 5
<210> 76
   <211> 9
   <212> PRT
   <213> Hepatitis C virus
<400> 76
   Trp Leu Arg Asp Val Trp Asp Trp Ile
   1               5
<210> 77
   <211> 9
   <212> PRT
   <213> Hepatitis C virus
<400> 77
   Tyr Val Val Leu Leu Phe Leu Leu Leu
   1               5
<210> 78
   <211> 9
   <212> PRT
   <213> Hepatitis C virus
<400> 78
   Leu Asn Pro Ser Val Ala Ala Thr Leu
   1               5
<210> 79
   <211> 9
   <212> PRT
   <213> Hepatitis C virus
<400> 79
   Tyr Val Tyr Asp His Leu Thr Pro Leu
   1               5
<210> 80
   <211> 9
   <212> PRT
   <213> Hepatitis C virus
<400> 80
   His Tyr Arg Asp Val Leu Lys Glu Met
   1               5
<210> 81
   <211> 9
   <212> PRT
   <213> Hepatitis C virus
<400> 81
   Thr Leu Arg Arg His Val Asp Leu Leu
   1               5
<210> 82
   <211> 9
   <212> PRT
   <213> Hepatitis C virus
<400> 82
   Ala Val Ile Pro Asp Arg Glu Val Leu
   1               5
<210> 83
   <211> 9
   <212> PRT
   <213> Hepatitis C virus
<400> 83
   Phe Leu Ile Ser Gln Leu Phe Thr Phe
   1               5
<210> 84
   <211> 9
   <212> PRT
   <213> Hepatitis C virus
<400> 84
   Val Ile Leu Asp Ser Phe Glu Pro Leu
   1               5
<210> 85
   <211> 9
   <212> PRT
   <213> Hepatitis C virus
<400> 85
   Ile Leu Pro Cys Ser Tyr Thr Thr Leu
   1               5
<210> 86
   <211> 9
   <212> PRT
   <213> Hepatitis C virus
<400> 86
   Gly Ile Leu Pro Phe Phe Met Phe Phe
   1 5
<210> 87
   <211> 9
   <212> PRT
   <213> Hepatitis C virus
<400> 87
   Pro Leu Leu Arg Asp Glu Val Thr Phe
   1 5
<210> 88
   <211> 9
   <212> PRT
   <213> Hepatitis C virus
<400> 88
   Thr Pro Pro Ala Val Pro Gln Thr Phe
   1 5
<210> 89
   <211> 9
   <212> PRT
   <213> Hepatitis C virus
<400> 89
   Phe Trp Ala Lys His Met Trp Asn Phe
   1 5
<210> 90
   <211> 9
   <212> PRT
   <213> Hepatitis C virus
<400> 90
   Thr Val Leu Ala Asp Phe Lys Thr Trp
   1 5
<210> 91
   <211> 9
   <212> PRT
   <213> Hepatitis C virus
<400> 91
   Ala Leu Tyr Asp Val Val Ser Thr Leu
   1 5
<210> 92
   <211> 9
   <212> PRT
   <213> Hepatitis C virus
<400> 92
   Trp His Tyr Pro Cys Thr Val Asn Phe
   1               5
<210> 93
   <211> 9
   <212> PRT
   <213> Hepatitis C virus
<400> 93
   Ala Thr Pro Pro Gly Ser Val Thr Phe
   1               5
<210> 94
   <211> 9
   <212> PRT
   <213> Hepatitis C virus
<400> 94
   Gly Leu Ile His Leu His Gln Asn Ile
   1               5
<210> 95
   <211> 9
   <212> PRT
   <213> Hepatitis C virus
<400> 95
   Ala Tyr Ser Gln Gln Thr Arg Gly Leu
   1               5
<210> 96
   <211> 9
   <212> PRT
   <213> Hepatitis C virus
<400> 96
   Ile Leu Met Thr His Phe Phe Ser Ile
   1               5
<210> 97
   <211> 9
   <212> PRT
   <213> Hepatitis C virus
<400> 97
   Phe Leu Ala Arg Leu Ile Trp Trp Leu
   1 5
<210> 98
   <211> 9
   <212> PRT
   <213> Hepatitis C virus
<400> 98
   Ala Phe Tyr Gly Val Trp Pro Leu Leu
   1 5
<210> 99
   <211> 9
   <212> PRT
   <213> Hepatitis C virus
<400> 99
   Phe Leu Ile Ser Gln Leu Phe Thr Phe
   1 5
<210> 100
   <211> 9
   <212> PRT
   <213> Hepatitis C virus
<400> 100
   Ile Leu Leu Gly Pro Ala Asp Ser Phe
   1 5
<210> 101
   <211> 9
   <212> PRT
   <213> Hepatitis C virus
<400> 101
   Ser Met Leu Thr Asp Pro Ser His Ile
   1 5
<210> 102
   <211> 9
   <212> PRT
   <213> Hepatitis C virus
<400> 102
   Ala Tyr Tyr Ser Met Val Gly Asn Trp
   1 5
<210> 103
   <211> 9
   <212> PRT
   <213> Hepatitis. C virus
<400> 103
   Gln Tyr Thr Leu Leu Phe Asn Ile Leu
   1 5
<210> 104
   <211> 9
   <212> PRT
   <213> Hepatitis C virus
<400> 104
   Leu Leu Lys Arg Leu His Gln Trp Ile
   1 5
<210> 105
   <211> 9
   <212> PRT
   <213> Hepatitis C virus
<400> 105
   Ser Gln Leu Asp Leu Ser Gly Trp Phe
   1 5
<210> 106
   <211> 9
   <212> PRT
   <213> Hepatitis C virus
<400> 106
   Trp Leu Arg Asp Val Trp Asp Trp Ile
   1 5
<210> 107
   <211> 9
   <212> PRT
   <213> Hepatitis C-virus
<400> 107
   Ile Thr Tyr Ser Thr Tyr Gly Lys Phe
   1 5
<210> 108
   <211> 9
   <212> PRT
   <213> Hepatitis C virus
<400> 108
   Ser Tyr Thr Trp Thr Gly Ala Leu Ile
   1               5
<210> 109
   <211> 9
   <212> PRT
   <213> Hepatitis C virus
<400> 109
   Leu Leu Ser Thr Thr Glu Trp Gln Ile
   1               5
<210> 110
   <211> 9
   <212> PRT
   <213> Hepatitis C virus
<400> 110
   Arg Tyr Ala Pro Ala Cys Lys Pro Leu
   1               5
<210> 111
   <211> 9
   <212> PRT
   <213> Hepatitis C virus
<400> 111
   Arg Leu Ile Trp Trp Leu Gln Tyr Phe
   1               5
<210> 112
   <211> 9
   <212> PRT
   <213> Hepatitis C virus
<400> 112
   Val Leu Ala Asp Phe Lys Thr Trp Leu
   1               5
<210> 113
   <211> 9
   <212> PRT
   <213> Hepatitis C virus
<400> 113.
   Leu Val Pro Gly Ala Ala Tyr Ala Phe
   1 5
<210> 114
   <211> 9
   <212> PRT
   <213> Hepatitis C virus
<400> 114
   Asp Leu Pro Gln Ile Ile Gln Arg Leu
   1 5
<210> 115
   <211> 9
   <212> PRT
   <213> Hepatitis C virus
<400> 115
   Trp Ile Cys Thr Val Leu Ala Asp Phe
   1 5
<210> 116
   <211> 9
   <212> PRT
   <213> Hepatitis C virus
<400> 116
   Phe Tyr Gly Val Trp Pro Leu Leu Leu
   1 5
<210> 117
   <211> 9
   <212> PRT
   <213> Hepatitis C virus
<400> 117
   Leu Leu Leu Ser Ile Leu Gly Pro Leu
   1 5
<210> 118
   <211> 9
   <212> PRT
   <213> Hepatitis C virus
<400> 118
   His Tyr Arg Asp Val Leu Lys Glu Met
   1 5
<210> 119
   <211> 9
   <212> PRT
   <213> Hepatitis C virus
<400> 119
   Leu Ile Trp Trp Leu Gln Tyr Phe Ile
   1 5
<210> 120
   <211> 9
   <212> PRT
   <213> Hepatitis C virus
<400> 120
   Ala Val Ile Pro Asp Arg Glu Val Leu
   1 5
<210> 121
   <211> 9
   <212> PRT
   <213> Hepatitis C virus
<400> 121
   Thr Arg Pro Pro His Gly Asn Trp Phe
   1 5
<210> 122
   <211> 9
   <212> PRT
   <213> Hepatitis C virus
<400> 122
   Lys Phe Pro Ala Ala Met Pro Val Trp
   1 5
<210> 123
   <211> 9
   <212> PRT
   <213> Hepatitis C virus
<400> 123
   Val Phe Pro Asp Leu Gly Val Arg Val
   1 5
<210> 124
   <211> 9
   <212> PRT
   <213> Hepatitis C virus
<400> 124
   Lys Leu Leu Pro Arg Leu Pro Gly Val
   1               5
<210> 125
   <211> 9
   <212> PRT
   <213> Hepatitis C virus
<400> 125
   Asp Met Pro Ser Thr Glu Asp Leu Val
   1               5
<210> 126
   <211> 9
   <212> PRT
   <213> Hepatitis C virus
<400> 126
   Tyr Leu Tyr Gly Ile Gly Ser Ala Val
   1               5
<210> 127
   <211> 9
   <212> PRT
   <213> Hepatitis C virus
<400> 127
   Tyr Leu Asn Thr Pro Gly Leu Pro Val
   1               5
<210> 128
   <211> 9
   <212> PRT
   <213> Hepatitis C virus
<400> 128
   Cys Leu Leu Leu Leu Ser Val Gly Val
   1               5
<210> 129
   <211> 9
   <212> PRT
   <213> Hepatitis C virus
<400> 129
   Leu Leu Leu Ser Val Gly Val Gly Ile
   1 5
<210> 130
   <211> 9
   <212> PRT
   <213> Hepatitis C virus
<400> 130
   Leu Leu Cys Pro Ser Gly His Val Val
   1 5
<210> 131
   <211> 9
   <212> :PRT
   <213> Hepatitis C virus
<400> 131
   Ala Ile Leu Ser Pro Gly Ala Leu Val
   1 5
<210> 132
   <211> 9
   <212> PRT
   <213> Hepatitis C virus
<400> 132
   Ser Leu Ile Arg Val Pro Tyr Phe Val
   1 5
<210> 133
   <211> 9
   <212> PRT
   <213> Hepatitis C virus
<400> 133
   Asp Val Trp Asp Trp Ile Cys Thr Val
   1 5
<210> 134
   <211> 9
   <212> PRT
   <213> Hepatitis C virus
<400> 134
   Val Ile Pro Ala Ser Gly Asp Val Val
   1 5
<210> 135
   <211> 9
   <212> PRT
   <213> Hepatitis C virus
<400> 135
   Arg Ala Leu Ala His Gly Val Arg Val
   1 5
<210> 136
   <211> 9
   <212> PRT
   <213> Hepatitis C virus
<400> 136
   Leu Ser Asp Gly Ser Trp Ser Thr Val
   1 5
<210> 137
   <211> 9
   <212> PRT
   <213> Hepatitis C virus
<400> 137
   Lys Leu Gln Asp Cys Thr Met Leu Val
   1 5
<210> 138
   <211> 9
   <212> PRT
   <213> Hepatitis C virus
<400> 138
   Tyr Cys Leu Thr Thr Gly Ser Val Val
   1 5
<210> 139
   <211> 9
   <212> PRT
   <213> Hepatitis C virus
<400> 139
   Ser Met Leu Thr Asp Pro Ser His Ile
   1 5
<210> 140
   <211> 9
   <212> PRT
   <213> Hepatitis C virus
<400> 140
   Ala Ala Phe Cys Ser Ala Met Tyr Val
   1 5
<210> 141
   <211> 9
   <212> PRT
   <213> Hepatitis C virus
<400> 141
   Tyr Ser Pro Gly Glu Ile Asn Arg Val
   1 5
<210> 142
   <211> 9
   <212> PRT
   <213> Hepatitis C virus
<400> 142
   Tyr Thr Asn Val Asp Gln Asp Leu Val
   1 5
<210> 143
   <211> 9
   <212> PRT
   <213> Hepatitis C virus
<400> 143
   Leu Arg Asp Glu Val Thr Phe Gln Val
   1 5
<210> 144
   <211> 9
   <212> PRT
   <213> Hepatitis C virus
<400> 144
   Leu Ala Ala Leu Thr Gly Thr Tyr Val
   1 5
<210> 145
   <211> 9
   <212> PRT
   <213> Hepatitis C virus
<400> 145
   Cys Glu Pro Glu Pro Asp Val Thr Val
   1               5
<210> 14.6
   <211> 9
   <212> PRT
   <213> Hepatitis C virus
<400> 146
<210> 147
   <211> 9
   <212> PRT
   <213> Hepatitis C virus
<400> 147
   Val Phe Pro Asp Leu Gly Val Arg Val
   1               5
<210> 148
   <211> 9
   <212> PRT
   <213> Hepatitis C virus
<400> 148
   Tyr Cys Phe Thr Pro Ser Pro Val Val
   1               5
<210> 149
   <211> 9
   <212> PRT
   <213> Hepatitis C virus
<400> 149
   Val Leu Gln Ala Ser Leu Ile Arg Val
   1               5
<210> 150
   <211> 9
   <212> PRT
   <213> Hepatitis C virus
<400> .150
<210> 151
   <211> 9
   <212> PRT
   <213> Hepatitis C virus
<400> 151
   Leu Leu Leu Ala Leu Pro Pro Arg Ala
   1               5
<210> 152
   <211> 9
   <212> PRT
   <213> Hepatitis C virus
<400> 152
   Val Leu Asp Asp His Tyr Arg Asp Val
   1               5
<210> 153
   <211> 9
   <212> PRT
   <213> Hepatitis C. virus
<400> 153
   Phe Ser Pro Arg Arg His Glu Thr Val
   1               5
<210> 154
   <211> 9
   <212> PRT
   <213> Hepatitis C virus
<400> 154
   Ser Val Ile Asp Cys Asn Thr Cys Val
   1               5
<210> 155
   <211> 9
   <212> PRT
   <213> Hepatitis C virus
<400> 155
   Gly Leu Ile Arg Ala Cys Thr Leu Val
   1               5
<210> 156
   <211> 9
   <212> PRT
   <213> Hepatitis C virus
<400> 156
   Thr Val Asn Phe Thr Ile Phe Lys Val
   1 5
<210> 15.7
   <211> 9
   <212> PRT
   <213> Hepatitis C virus
<400> 157
<210> 158
   <211> 9
   <212> PRT
   <213> Hepatitis C virus
<400> 158
   Pro Leu Leu Arg His His Asn Met Val
   1 5
<210> 159
   <211> 9
   <212> PRT
   <213> Hepatitis C virus
<400> 159
   Gln Leu Asp Leu Ser Gly Trp Phe Val
   1 5
<210> 160
   <211> 9
   <212> PRT
   <213> Hepatitis C virus
<400> 160
   Thr Leu Ala Ala Arg Asn Ala Ser Val
   1 5
<210> 161
   <211> 9
   <212> PRT
   <213> Hepatitis C virus
<400> 161
   Arg Leu Gly Ala Val Gln Asn Glu Val
   1 5
<210> 162
   <211> 9
   <212> PRT
   <213> Hepatitis C. virus
<400> 162
   Val Ile Leu Asp Ser Phe Glu Pro Leu
   1 5
<210> 163
   <211> 9
   <212> PRT
   <213> Hepatitis C virus
<400> 163
   Ala Ala Leu Glu Asn Leu Val Val Leu
   1 5
<210> 164
   <211> 9
   <212> PRT
   <213> Hepatitis C virus
<400> 164
   Leu Leu Glu Asp Thr Asp Thr Pro Ile
   1 5
<210> 165
   <211> 9
   <212> PRT
   <213> Hepatitis C virus
<400> 165
   Val Val Thr Ser Thr Trp Val Leu Val
   1 5
<210> 166
   <211> 9
   <212> PRT
   <213> Hepatitis C virus
<400> 166
   Phe Ser Leu Asp Pro Thr Phe Thr Ile
   1 5
<210> 167
   <211> 9
   <212> PRT
   <213> Hepatitis C virus
<400> 167
   Thr Ile Pro Ala Ser Ala Tyr Glu Val
   1 5
<210> 168
   <211> 9
   <212> PRT
   <213> Hepatitis C virus
<400> 168
   Asp Leu Leu Glu Asp Thr Asp Thr Pro
   1 5
<210> 169
   <211> 9
   <212> PRT
   <213> Hepatitis C virus
<400> 169
   Leu Leu Leu Ser Ile Leu Gly Pro Leu
   1 5
<210> 170
   <211> 9
   <212> PRT
   <213> Hepatitis C virus
<400> 170
   Val Leu Ala Asp Phe Lys Thr Trp Leu
   1 5
<210> 171
   <211> 9
   <212> PRT
   <213> Hepatitis C virus
<400> 171
   Ser Ile Leu Gly Ile Gly Thr Val Leu
   1 5
<210> 172
   <211> 9
   <212> PRT
   <213> Hepatitis C virus
<400> 172
   Ala Gly Asp Asn Phe Pro Tyr Leu Val
   1 5
<210> 173
   <211> 9
   <212> PRT
   <213> Hepatitis C virus
<400> 173
   Ile Leu Pro Cys Ser Tyr Thr Thr Leu
   1 5
<210>. 174
   <211> 9
   <212> PRT
   <213> Hepatitis C virus
<400> 174
   Val Ala Ala Glu Glu Tyr Val Glu Val
   1 5
<210> 175
   <211> 9.
   <212> PRT
   <213> Hepatitis C virus
<400> 175
   Leu Ala Val Ala Val Glu Pro Val Val
   1 5
<210> 176
   <211> 9
   <212> PRT
   <213>. Hepatitis C virus
<400> 176
   Ala Leu Tyr Asp Val Val Ser Thr Leu
   1 5
<210> 177
   <211> 9
   <212> PRT
   <213> Hepatitis C virus
<400> 177
   Phe Leu Ala Arg Leu Ile Trp Trp Leu
   1 5
<210> 178
   <211> 9
   <212> PRT
   <213> Hepatitis C virus
<400> 178
   Arg Leu Leu Ala Pro Ile Thr Ala Tyr
   1 5
<210> 179
   <211> 9
   <212> PRT
   <213> Hepatitis C virus
<400> 179
   Trp Leu Arg Asp Val Trp Asp Trp Ile
   1 5
<210> 180
   <211> 9
   <212> PRT
   <213> Hepatitis C virus
<400> 180
   Cys Val Asn Gly Ala Cys Trp Thr Val
   1 5
<210> 181
   <211> 9
   <212> PRT
   <213> Hepatitis C virus
<400> 181
   Tyr Val Tyr Asp His Leu Thr Pro Leu
   1 5
<210> 182
   <211> 9
   <212> PRT
   <213> Hepatitis C virus
<400> 182
   Thr Val Val Leu Thr Glu Ser Thr Val
   1 5
<210> 183
   <211> 9
   <212> PRT
   <213> Hepatitis C virus
<400> 183
   Ala Ala Arg Ala Leu Ala His Gly Val
   1 5
<210> 184
   <211> 3010
   <212> PRT
   <213> Hepatitis C virus
<400> 184
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Thr Trp Ala Gln Pro Gly
   65 70 75 80
   Tyr Pro Trp Pro Leu Tyr Gly Asn Glu Gly Met Gly Trp Ala Gly Trp
   85 90 95
   Leu Leu Ser Pro Arg Gly Ser Arg Pro Ser Trp Gly Pro Thr Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
   130 135 140
   Gly Gly Ala Ala Arg Ala Leu Ala His Gly Val Arg Val Leu Glu Asp
   145 150 155 160
   Gly Val Asn Tyr Ala Thr Gly Asn Leu Pro Gly Cys Ser Phe Ser Ile
   165 170 175
   Phe Leu Leu Ala Leu Leu Ser Cys Leu Thr Ile Pro Ala Ser Ala Tyr
   180 185 190
   Glu Val Arg Asn Val Ser Gly Ile Tyr His Val Thr Asn Asp Cys Ser
   195 200 205
   Asn Ser Ser Ile Val Tyr Glu Ala Ala Asp Met Ile Met His Thr Pro
   210 215 220
   Gly Cys Val Pro Cys Val Arg Glu Ser Asn Phe Ser Arg Cys Trp Val
   225 230 235 240
   Ala Leu Thr Pro Thr Leu Ala Ala Arg Asn Ser Ser Ile Pro Thr Thr
   245 250 255
   Thr Ile Arg Arg His Val Asp Leu Leu Val Gly Ala Ala Ala Leu Cys
   260 265 270
   Ser Ala Met Tyr Val Gly Asp Leu Cys Gly Ser Val Phe Leu Val Ser
   275 280 285
   Gln Leu Phe Thr Phe Ser Pro Arg Arg Tyr Glu Thr Val Gln Asp Cys
   290 295 300
   Asn Cys Ser Ile Tyr Pro Gly His Val Ser Gly His Arg Met Ala Trp
   305 310 315 320
   Asp Met Met Met Asn Trp Ser Pro Thr Thr Ala Leu Val Val Ser Gln
   325 330 335
   Leu Leu Arg Ile Pro Gln Ala Val Val Asp Met Val Ala Gly Ala His
   340 345 350
   Trp Gly Val Leu Ala Gly Leu Ala Tyr Tyr Ser Met Val Gly Asn Trp
   355 360 365
   Ala Lys Val Leu Ile Val Met Leu Leu Phe Ala Gly Val Asp Gly His
   370 375 380
   Thr His Val Thr Gly Gly Arg Val Ala Ser Ser Thr Gln Ser Leu Val
   385 390 395 400
   Ser Trp Leu Ser Gln Gly Pro Ser Gln Lys Ile Gln Leu Val Asn Thr
   405 410 415
   Asn Gly Ser Trp His Ile Asn Arg Thr Ala Leu Asn Cys Asn Asp Ser
   420 425 430
   Leu Gln Thr Gly Phe Ile Ala Ala Leu Phe Tyr Ala His Arg Phe Asn
   435 440 445
   Ala Ser Gly Cys Pro Glu Arg Met Ala Ser Cys Arg Pro Ile Asp Glu
   450 455 460
   Phe Ala Gln Gly Trp Gly Pro Ile Thr His Asp Met Pro Glu Ser Ser
   465 470 475 480
   Asp Gln Arg Pro Tyr Cys Trp His Tyr Ala Pro Arg Pro Cys Gly Ile
   485 490 495
   Val Pro Ala Ser Gln Val Cys Gly Pro Val Tyr Cys Phe Thr Pro Ser
   500 505 510
   Pro Val Val Val Gly Thr Thr Asp Arg Phe Gly Ala Pro Thr Tyr Ser
   515 520 525
   Trp Gly Glu Asn Glu Thr Asp Val Leu Leu Leu Ser Asn Thr Arg Pro
   530 535 540
   Pro Gln Gly Asn Trp Phe Gly Cys Thr Trp Met Asn Ser Thr Gly Phe
   545 550 555 560
   Thr Lys Thr Cys Gly Gly Pro Pro Cys Asn Ile Gly Gly Val Gly Asn
   565 570 575
   Asn Thr Leu Val Cys Pro Thr Asp Cys Phe Arg Lys His Pro Glu Ala
   580 585 590
   Thr Tyr Thr Lys Cys Gly Ser Gly Pro Trp Leu Thr Pro Arg Cys Met
   595 600 605
   Val Asp Tyr Pro Tyr Arg Leu Trp His Tyr Pro Cys Thr Val Asn Phe
   610 615 620
   Thr Val Phe Lys Val Arg Met Tyr Val Gly Gly Val Glu His Arg Leu
   625 630 635 640
   Asn Ala Ala Cys Asn Trp Thr Arg Gly Glu Arg Cys Asp Leu Glu Asp
   645 650 655
   Arg Asp Arg Ser Glu Leu Ser Pro Leu Leu Leu Ser Thr Thr Glu Trp
   660 665 670
   Gln Ile Leu Pro Cys Ser Phe Thr Thr Leu Pro Ala Leu Ser Thr Gly
   675 680 685
   Leu Ile His Leu His Arg Asn Ile Val Asp Val Gln Tyr Leu Tyr Gly
   690 695 700
   Ile Gly Ser Ala Val Val Ser Phe Ala Ile Lys Trp Glu Tyr Ile Leu
   705 710 715 720
   Leu Leu Phe Leu Leu Leu Ala Asp Ala Arg Val Cys Ala Cys Leu Trp
   725 730 735
   Met Met Leu Leu Ile Ala Gln Ala Glu Ala Thr Leu Glu Asn Leu Val
   740 745 750
   Val Leu Asn Ala Ala Ser Val Ala Gly Ala His Gly Leu Leu Ser Phe
   755 760 765
   Leu Val Phe Phe Cys Ala Ala Trp Tyr Ile Lys Gly Arg Leu Val Pro
   770 775 780
   Gly Ala Ala Tyr Ala Leu Tyr Gly Val Trp Pro Leu Leu Leu Leu Leu
   785 790 795 800
   Leu Ala Leu Pro Pro Arg Ala Tyr Ala Met Asp Arg Glu Met Ala Ala
   805 810 815
   Ser Cys Gly Gly Ala Val Phe Val Gly Leu Val Leu Leu Thr Leu Ser
   820 825 830
   Pro Tyr Tyr Lys Val Phe Leu Ala Arg Leu Ile Trp Trp Leu Gln Tyr
   835 840 845
   Phe Ile Thr Arg Ala Glu Ala His Leu Gln Val Trp Val Pro Pro Leu
   850 855 860
   Asn Val Arg Gly Gly Arg Asp Ala Ile Ile Leu Leu Thr Cys Ala Val
   865 870 875 880
   His Pro Glu Leu Ile Phe Asp Ile Thr Lys Leu Leu Leu Ala Ile Leu
   885 890 895
   Gly Pro Leu Met Val Leu Gln Ala Gly Ile Thr Arg Val Pro Tyr Phe
   900 905 910
   Val Arg Ala Gln Gly Leu Ile Arg Ala Cys Met Leu Val Arg Lys Val
   915 920 925
   Ala Gly Gly His Tyr Val Gln Met Ala Phe Met Lys Leu Ala Ala Leu
   930 935 940
   Thr Gly Thr Tyr Val Tyr Asp His Leu Thr Pro Leu Arg Asp Trp Ala
   945 950 955 960
   His Ala Gly Leu Arg Asp Leu Ala Val Ala Val Glu Pro Val Val Phe
   965 970 975
   Ser Asp Met Glu Thr Lys Leu Ile Thr Trp Gly Ala Asp Thr Ala Ala
   980 985 990
   Cys Gly Asp Ile Ile Ser Gly Leu Pro Val Ser Ala Arg Arg Gly Lys
   995 1000 1005
   Glu Ile Leu Leu Gly Pro Ala Asp Ser Phe Gly Glu Gln Gly Trp
   1010 1015 1020
   Arg Leu Leu Ala Pro Ile Thr Ala Tyr Ser Gln Gln Thr Arg Gly
   1025 1030 1035
   Leu Leu Gly Cys Ile Ile Thr Ser Leu Thr Gly Arg Asp Lys Asn
   1040 1045 1050
   Gln Val Asp Gly Glu Val Gln Val Leu Ser Thr Ala Thr Gln Ser
   1055 1060 1065
   Phe Leu Ala Thr Cys Val Asn Gly Val Cys Trp Thr Val Tyr His
   1070 1075 1080
   Gly Ala Gly Ser Lys Thr Leu Ala Gly Pro Lys Gly Pro Ile Thr
   1085 1090 1095
   Gln Met Tyr Thr Asn Val Asp Gln Asp Leu Val Gly Trp Pro Ala
   1100 1105 1110
   Pro Pro Gly Ala Arg Ser Met Thr Pro Cys Thr Cys Gly Ser Ser
   1115 1120 1125
   Asp Leu Tyr Leu Val Thr Arg His Ala Asp Val Val Pro Val Arg
   1130 1135 1140
   Arg Arg Gly Asp Ser Arg Gly Ser Leu Leu Ser Pro Arg Pro Ile
   1145 1150 1155
   Ser Tyr Leu Lys Gly Ser Ser Gly Gly Pro Leu Leu Cys Pro Ser
   1160 1165 1170
   Gly His Val Val Gly Ile Phe Arg Ala Ala Val Cys Thr Arg Gly
   1175 1180 1185
   Val Ala Lys Ala Val Asp Phe Ile Pro Val Glu Ser Met Glu Thr
   1190 1195 1200
   Thr Met Arg Ser Pro Val Phe Thr Asp Asn Ser Ser Pro Pro Ala
   1205 1210 1215
   Val Pro Gln Thr Phe Gln Val Ala His Leu His Ala Pro Thr Gly
   1220 1225 1230
   Ser Gly Lys Ser Thr Lys Val Pro Ala Ala Tyr Ala Ala Gln Gly
   1235 1240 1245
   Tyr Lys Val Leu Val Leu Asn Pro Ser Val Ala Ala Thr Leu Gly
   1250 1255 1260
   Phe Gly Ala Tyr Met Ser Lys Ala His Gly Ile Glu Pro Asn Ile
   1265 1270 1275
   Arg Thr Gly Val Arg Thr Ile Thr Thr Gly Gly Pro Ile Thr Tyr
   1280 1285 1290
   Ser Thr Tyr Cys Lys Phe Leu Ala Asp Gly Gly Cys Ser Gly Gly
   1295 1300 1305
   Ala Tyr Asp Ile Ile Ile Cys Asp Glu Cys His Ser Thr Asp Ser
   1310 1315 1320
   Thr Thr Ile Leu Gly Ile Gly Thr Val Leu Asp Gln Ala Glu Thr
   1325 1330 1335
   Ala Gly Ala Arg Leu Val Val Leu Ala Thr Ala Thr Pro Pro Gly
   1340 1345 1350
   Ser Ile Thr Val Pro His Pro Asn Ile Glu Glu Val Ala Leu Ser
   1355 1360 1365
   Asn Thr Gly Glu Ile Pro Phe Tyr Gly Lys Ala Ile Pro Ile Glu
   1370 1375 1380
   Ala Ile Lys Gly Gly Arg His Leu Ile Phe Cys His Ser Lys Lys
   1385 1390 1395
   Lys Cys Asp Glu Leu Ala Ala Lys Leu Thr Gly Leu Gly Leu Asn
   1400 1405 1410
   Ala Val Ala Tyr Tyr Arg Gly Leu Asp Val Ser Val Ile Pro Thr
   1415 1420 1425
   Ser Gly Asp Val Val Val Val Ala Thr Asp Ala Leu Met Thr Gly
   1430 1435 1440
   Phe Thr Gly Asp Phe Asp Ser Val Ile Asp Cys Asn Thr Cys Val
       1445                    1450                    1455
   Thr Gln Thr Val Asp Phe Ser Leu Asp Pro Thr Phe Thr Ile Glu
       1460                    1465                    1470
   Thr Thr Thr Leu Pro Gln Asp Ala Val Ser Arg Ala Gln Arg Arg
       1475                    1480                    1485
   Gly Arg Thr Gly Arg Gly Arg Ser Gly Ile Tyr Arg Phe Val Thr
       1490                    1495                    1500
   Pro Gly Glu Arg Pro Ser Gly Met Phe Asp Ser Ser Val Leu Cys
       1505                    1510                    1515
   Glu Cys Tyr Asp Ala Gly Cys Ala Trp Tyr Glu Leu Thr Pro Ala
       1520                    1525                    1530
   Glu Thr Ser Val Arg Leu Arg Ala Tyr Leu Asn Thr Pro Gly Leu
       1535                    1540                    1545
   Pro Val Cys Gln Asp His Leu Glu Phe Trp Glu Ser Val Phe Thr
       1550                    1555                    1560
   Gly Leu Thr His Ile Asp Ala His Phe Leu Ser Gln Thr Lys Gln
       1565                    1570                    1575
   Ala Gly Asp Asn Leu Pro Tyr Leu Val Ala Tyr Gln Ala Thr Val
       1580                    1585                    1590
   Cys Ala Arg Ala Gln Ala Pro Pro Pro Ser Trp Asp Gln Met Trp
       1595                    1600                    1605
   Lys Cys Leu Ile Arg Leu Lys Pro Thr Leu His Gly Pro Thr Pro
       1610                    1615                    1620
   Leu Leu Tyr Arg Leu Gly Ala Val Gln Asn Glu Val Thr Leu Thr
       1625                    1630                    1635
   His Pro Ile Thr Lys Tyr Ile Met Ala Cys Met Ser Ala Asp Leu
       1640                    1645                    1650
   Glu Val Val Thr Ser Thr Trp Val Leu Val Gly Gly Val Leu Ala
       1655                    1660                    1665
   Ala Leu Ala Ala Tyr Cys Leu Thr Thr Gly Ser Val Val Ile Val
   1670 1675 1680
   Gly Arg Ile Ile Leu Ser Gly Arg Pro Ala Val Ile Pro Asp Arg
   1685 1690 1695
   Glu Val Leu Tyr Gln Glu Phe Asp Glu Met Glu Glu Cys Ala Ser
   1700 1705 1710
   His Leu Pro Tyr Ile Glu Gln Gly Met Gln Leu Ala Glu Gln Phe
   1715 1720 1725
   Lys Gln Lys Ala Leu Gly Leu Leu Gln Thr Ala Thr Lys Gln Ala
   1730 1735 1740
   Glu Ala Ala Ala Pro Val Val Glu Ser Lys Trp Arg Ala Leu Glu
   1745 1750 1755
   Val Phe Trp Ala Lys His Met Trp Asn Phe Ile Ser Gly Ile Gln
   1760 1765 1770
   Tyr Leu Ala Gly Leu Ser Thr Leu Pro Gly Asn Pro Ala Ile Ala
   1775 1780 1785
   Ser Leu Met Ala Phe Thr Ala Ser Ile Thr Ser Pro Leu Thr Thr
   1790 1795 1800
   Gln Asn Thr Leu Leu Phe Asn Ile Leu Gly Gly Trp Val Ala Ala
   1805 1810 1815
   Gln Leu Ala Pro Pro Ser Ala Ala Ser Ala Phe Val Gly Ala Gly
   1820 1825 1830
   Ile Ala Gly Ala Ala Val Gly Ser Ile Gly Leu Gly Lys Val Leu
   1835 1840 1845
   Val Asp Ile Leu Ala Gly Tyr Gly Ala Gly Val Ala Gly Ala Leu
   1850 1855 1860
   Val Ala Phe Lys Val Met Ser Gly Glu Met Pro Ser Thr Glu Asp
   1865 1870 1875
   Leu Val Asn Leu Leu Pro Ala Ile Leu Ser Pro Gly Ala Leu Val
   1880 1885 1890
   Val Gly Val Val Cys Ala Ala Ile Leu Arg Arg His Val Gly Pro
   1895 1900 1905
   Gly Glu Gly Ala Val Gln Trp Met Asn Arg Leu Ile Ala Phe Ala
   1910 1915 1920
   Ser Arg Gly Asn His Val Ser Pro Thr His Tyr Val Pro Glu Ser
   1925 1930 1935
   Asp Ala Ala Ala Arg Val Thr Gln Ile Leu Ser Ser Leu Thr Ile
   1940 1945 1950
   Thr Gln Leu Leu Lys Arg Leu His Gln Trp Ile Asn Glu Asp Cys
   1955 1960 1965
   Ser Thr Pro Cys Ser Gly Ser Trp Leu Lys Asp Val Trp Asp Trp
   1970 1975 1980
   Ile Cys Thr Val Leu Ser Asp Phe Lys Thr Trp Leu Gln Ser Lys
   1985 1990 1995
   Leu Leu Pro Arg Leu Pro Gly Leu Pro Phe Leu Ser Cys Gln Arg
   2000 2005 2010
   Gly Tyr Lys Gly Val Trp Arg Gly Asp Gly Ile Met Gln Thr Thr
   2015 2020 2025
   Cys Pro Cys Gly Ala Gln Ile Thr Gly His Val Lys Asn Gly Ser
   2030 2035 2040
   Met Arg Ile Val Gly Pro Lys Thr Cys Ser Asn Thr Trp His Gly
   2045 2050 2055
   Thr Phe Pro Ile Asn Ala Tyr Thr Thr Gly Pro Cys Thr Pro Ser
   2060 2065 2070
   Pro Ala Pro Asn Tyr Ser Arg Ala Leu Trp Arg Val Ala Ala Glu
   2075 2080 2085
   Glu Tyr Val Glu Val Thr Arg Val Gly Asp Phe His Tyr Val Thr
   2090 2095 2100
   Gly Met Thr Thr Asp Asn Val Lys Cys Pro Cys Gln Val Pro Ala
   2105 2110 2115
   Pro Glu Phe Phe Thr Glu Val Asp Gly Val Arg Leu His Arg Tyr
   2120 2125 2130
   Ala Pro Val Cys Lys Pro Leu Leu Arg Glu Glu Val Val Phe Gln
   2135 2140 2145
   Val Gly Leu Asn Gln Tyr Leu Val Gly Ser Gln Leu Pro Cys Glu
   2150 2155 2160
   Pro Glu Pro Asp Val Ala Val Leu Thr Ser Met Leu Thr Asp Pro
   2165 2170 2175
   Ser His Ile Thr Ala Glu Thr Ala Lys Arg Arg Leu Ala Arg Gly
   2180 2185 2190
   Ser Pro Pro Ser Leu Ala Ser Ser Ser Ala Ser Gln Leu Ser Ala
   2195 2200 2205
   Pro Ser Leu Lys Ala Thr Cys Thr Thr His His Asp Ser Pro Asp
   2210 2215 2220
   Ala Asp Leu Ile Glu Ala Asn Leu Leu Trp Arg Gln Glu Met Gly
   2225 2230 2235
   Gly Asn Ile Thr Arg Val Glu Ser Glu Asn Lys Val Val Ile Leu
   2240 2245 2250
   Asp Ser Phe Asp Pro Ile Arg Ala Val Glu Asp Glu Arg Glu Ile
   2255 2260 2265
   Ser Val Pro Ala Glu Ile Leu Arg Lys Pro Arg Lys Phe Pro Pro
   2270 2275 2280
   Ala Leu Pro Ile Trp Ala Arg Pro Asp Tyr Asn Pro Pro Leu Leu
   2285 2290 2295
   Glu Ser Trp Lys Asp Pro Asp Tyr Val Pro Pro Val Val His Gly
   2300 2305 2310
   Cys Pro Leu Pro Ser Thr Lys Ala Pro Pro Ile Pro Pro Pro Arg
   2315 2320 2325
   Arg Lys Arg Thr Val Val Leu Thr Glu Ser Thr Val Ser Ser Ala
   2330 2335 2340
   Leu Ala Glu Leu Ala Thr Lys Thr Phe Gly Ser Ser Gly Ser Ser
   2345 2350 2355
   Ala Val Asp Ser Gly Thr Ala Thr Gly Pro Pro Asp Gln Ala Ser
   2360 2365 2370
   Asp Asp Gly Asp Lys Gly Ser Asp Val Glu Ser Tyr Ser Ser Met
   2375 2380 2385
   Pro Pro Leu Glu Gly Glu Pro Gly Asp Pro Asp Leu Ser Asp Gly
   2390 2395 2400
   Ser Trp Ser Thr Val Ser Gly Glu Ala Gly Glu Asp Val Val Cys
   2405 2410 2415
   Cys Ser Met Ser Tyr Thr Trp Thr Gly Ala Leu Ile Thr Pro Cys
   2420 2425 2430
   Ala Ala Glu Glu Ser Lys Leu Pro Ile Asn Pro Leu Ser Asn Ser
   2435 2440 2445
   Leu Leu Arg His His Ser Met Val Tyr Ser Thr Thr Ser Arg Ser
   2450 2455 2460
   Ala Ser Leu Arg Gln Lys Lys Val Thr Phe Asp Arg Leu Gln Val
   2465 2470 2475
   Leu Asp Asp His Tyr Arg Asp Val Leu Lys Glu Met Lys Ala Lys
   2480 2485 2490
   Ala Ser Thr Val Lys Ala Arg Leu Leu Ser Ile Glu Glu Ala Cys
   2495 2500 2505
   Lys Leu Thr Pro Pro His Ser Ala Lys Ser Lys Phe Gly Tyr Gly
   2510 2515 2520
   Ala Lys Asp Val Arg Ser Leu Ser Ser Arg Ala Val Asn His Ile
   2525 2530 2535
   Arg Ser Val Trp Glu Asp Leu Leu Glu Asp Thr Glu Thr Pro Ile
   2540 2545 2550
   Asp Thr Thr Ile Met Ala Lys Asn Glu Val Phe Cys Val Gln Pro
   2555 2560 2565
   Glu Lys Gly Gly Arg Lys Pro Ala Arg Leu Ile Val Phe Pro Asp
   2570 2575 2580
   Leu Gly Val Arg Val Cys Glu Lys Met Ala Leu Tyr Asp Val Val
   2585 2590 2595
   Ser Thr Leu Pro Gln Ala Val Met Gly Pro Ser Tyr Gly Phe Gln
   2600 2605 2610
   Tyr Ser Pro Gly Gln Arg Val Glu Phe Leu Val Asn Thr Trp Lys
   2615 2620 2625
   Ser Lys Lys Cys Pro Met Gly Phe Ser Tyr Asp Thr Arg Cys Phe
   2630 2635 2640
   Asp Ser Thr Val Thr Glu Asn Asp Ile Arg Thr Glu Glu Ser Ile
   2645 2650 2655
   Tyr Gln Cys Cys Asp Leu Ala Pro Glu Ala Arg Gln Ala Ile Arg
   2660 2665 2670
   Ser Leu Thr Glu Arg Leu Tyr Val Gly Gly Pro Leu Thr Asn Ser
   2675 2680 2685
   Lys Gly Gln Asn Cys Gly Tyr Arg Arg Cys Arg Ala Ser Gly Val
   2690 2695 2700
   Leu Thr Thr Ser Cys Gly Asn Thr Leu Thr Cys Tyr Leu Lys Ala
   2705 2710 2715
   Thr Ala Ala Cys Arg Ala Ala Lys Leu Gln Asp Cys Thr Met Leu
   2720 2725 2730
   Val Asn Gly Asp Asp Leu Val Val Ile Cys Glu Ser Ala Gly Thr
   2735 2740 2745
   Gln Glu Asp Ala Ala Ala Leu Arg Ala Phe Thr Glu Ala Met Thr
   2750 2755 2760
   Arg Tyr Ser Ala Pro Pro Gly Asp Pro Pro Gln Pro Glu Tyr Asp
   2765 2770 2775
   Leu Glu Leu Ile Thr Ser Cys Ser Ser Asn Val Ser Val Ala His
   2780 2785 2790
   Asp Ala Ser Gly Lys Arg Val Tyr Tyr Leu Thr Arg Asp Pro Thr
   2795 2800 2805
   Thr Pro Leu Ala Arg Ala Ala Trp Glu Thr Val Arg His Thr Pro
   2810 2815 2820
   Val Asn Ser Trp Leu Gly Asn Ile Ile Met Tyr Ala Pro Thr Leu
   2825 2830 2835
   Trp Ala Arg Met Ile Leu Met Thr His Phe Phe Ser Ile Leu Leu
   2840 2845 2850
   Ala Gln Glu Gln Leu Glu Lys Ala Leu Asp Cys Gln Ile Tyr Gly
   2855 2860 2865
   Ala Cys Tyr Ser Ile Glu Pro Leu Asp Leu Pro Gln Ile Ile Glu
   2870 2875 2880
   Arg Leu His Gly Leu Ser Ala Phe Ser Leu His Ser Tyr Ser Pro
   2885 2890 2895
   Gly Glu Ile Asn Arg Val Ala Ser Cys Leu Arg Lys Leu Gly Val
   2900 2905 2910
   Pro Pro Leu Arg Val Trp Arg His Arg Ala Arg Ser Val Arg Ala
   2915 2920 2925
   Lys Leu Leu Ser Gln Gly Gly Arg Ala Ala Thr Cys Gly Lys Tyr
   2930 2935 2940
   Leu Phe Asn Trp Ala Val Lys Thr Lys Leu Lys Leu Thr Pro Ile
   2945 2950 2955
   Pro Ala Ala Ser Gln Leu Asp Leu Ser Gly Trp Phe Val Ala Gly
   2960 2965 2970
   Tyr Asn Gly Gly Asp Ile Tyr His Ser Leu Ser Arg Ala Arg Pro
   2975 2980 2985
   Arg Trp Phe Met Leu Cys Leu Leu Leu Leu Ser Val Gly Val Gly
   2990 2995 3000
   Ile Tyr Leu Leu Pro Asn Arg
   3005 3010
<210> 185
   <211> 3010
   <212> PRT
   <213> Hepatitis C virus
<400> 185
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Asn Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Ile Pro Ala Ser Ala Tyr
               180                 185                 190
   Glu Val Arg Asn Val Ser Gly Ile Tyr His Val Thr Asn Asp Cys Ser
           195                 200                 205
   Asn Ser Ser Ile Val Tyr Glu Ala Ala Asp Val Ile Met His Ala Pro
   210 215 220
   Gly Cys Val Pro Cys Val Arg Glu Asn Asn Ser Ser Arg Cys Trp Val
   225 230 235 240
   Ala Leu Thr Pro Thr Leu Ala Ala Arg Asn Ala Ser Val Pro Thr Thr
   245 250 255
   Thr Leu Arg Arg His Val Asp Leu Leu Val Gly Thr Ala Ala Phe Cys
   260 265 270
   Ser Ala Met Tyr Val Gly Asp Leu Cys Gly Ser Val Phe Leu Ile Ser
   275 280 285
   Gln Leu Phe Thr Phe Ser Pro Arg Arg His Glu Thr Val Gln Asp Cys
   290 295 300
   Asn Cys Ser Ile Tyr Pro Gly His Val Ser Gly His Arg Met Ala Trp
   305 310 315 320
   Asp Met Met Met Asn Trp Ser Pro Thr Ala Ala Leu Val Val Ser Gln
   325 330 335
   Leu Leu Arg Ile Pro Gln Ala Val Met Asp Met Val Ala Gly Ala His
   340 345 350
   Trp Gly Val Leu Ala Gly Leu Ala Tyr Tyr Ser Met Val Gly Asn Trp
   355 360 365
   Ala Lys Val Leu Ile Val Met Leu Leu Phe Ala Gly Val Asp Gly His
   370 375 380
   Thr Arg Val Thr Gly Gly Val Gln Gly His Val Thr Ser Thr Leu Thr
   385 390 395 400
   Ser Leu Phe Arg Pro Gly Ala Ser Gln Lys Ile Gln Leu Val Asn Thr
   405 410 415
   Asn Gly Ser Trp His Ile Asn Arg Thr Ala Leu Asn Cys Asn Asp Ser
   420 425 430
   Leu Lys Thr Gly Phe Leu Ala Ala Leu Phe Tyr Thr His Lys Phe Asn
   435 440 445
   Ala Ser Gly Cys Pro Glu Arg Met Ala Ser Cys Arg Ser Ile Asp Lys
   450 455 460
   Phe Asp Gln Gly Trp Gly Pro Ile Thr Tyr Ala Gln Pro Asp Asn Ser
   465 470 475 480
   Asp Gln Arg Pro Tyr Cys Trp His Tyr Ala Pro Arg Gln Cys Gly Ile
   485 490 495
   Val Pro Ala Ser Gln Val Cys Gly Pro Val Tyr Cys Phe Thr Pro Ser
   500 505 510
   Pro Val Val Val Gly Thr Thr Asp Arg Phe Gly Ala Pro Thr Tyr Asn
   515 520 525
   Trp Gly Asp Asn Glu Thr Asp Val Leu Leu Leu Asn Asn Thr Arg Pro
   530 535 540
   Pro His Gly Asn Trp Phe Gly Cys Thr Trp Met Asn Ser Thr Gly Phe
   545 550 555 560
   Thr Lys Thr Cys Gly Gly Pro Pro Cys Asn Ile Arg Gly Val Gly Asn
   565 570 575
   Asn Thr Leu Thr Cys Pro Thr Asp Cys Phe Arg Lys His Pro Asp Ala
   580 585 590
   Thr Tyr Thr Lys Cys Gly Ser Gly Pro Trp Leu Thr Pro Arg Cys Leu
   595 600 605
   Val Asp Tyr Pro Tyr Arg Leu Trp His Tyr Pro Cys Thr Val Asn Phe
   610 615 620
   Thr Ile Phe Lys Val Arg Met Tyr Val Gly Gly Val Glu His Arg Leu
   625 630 635 640
   Asp Ala Ala Cys Asn Trp Thr Arg Gly Glu Arg Cys Asp Leu Glu Asp
   645 650 655
   Arg Asp Arg Ala Glu Leu Ser Pro Leu Leu Leu Ser Thr Thr Glu Trp
   660 665 670
   Gln Ile Leu Pro Cys Ser Tyr Thr Thr Leu Pro Ala Leu Ser Thr Gly
   675 680 685
   Leu Ile His Leu His Gln Asn Ile Val Asp Ile Gln Tyr Leu Tyr Gly
   690 695 700
   Ile Gly Ser Ala Val Val Ser Ile Ala Ile Lys Trp Glu Tyr Val Val
   705 710 715 720
   Leu Leu Phe Leu Leu Leu Ala Asp Ala Arg Val Cys Ala Cys Leu Trp
   725 730 735
   Met Met Leu Leu Ile Ala Gln Ala Glu Ala Ala Leu Glu Asn Leu Val
   740 745 750
   Val Leu Asn Ala Ala Ser Val Val Gly Ala His Gly Met Leu Pro Phe
   755 760 765
   Phe Met Phe Phe Cys Ala Ala Trp Tyr Met Lys Gly Arg Leu Val Pro
   770 775 780
   Gly Ala Ala Tyr Ala Phe Tyr Gly Val Trp Pro Leu Leu Leu Leu Leu
   785 790 795 800
   Leu Ala Leu Pro Pro Arg Ala Tyr Ala Met Asp Arg Glu Met Val Ala
   805 810 815
   Ser Cys Gly Gly Gly Val Phe Val Gly Leu Ala Leu Leu Thr Leu Ser
   820 825 830
   Pro Tyr Cys Lys Val Phe Leu Ala Arg Leu Ile Trp Trp Leu Gln Tyr
   835 840 845
   Phe Ile Thr Lys Ala Glu Ala His Leu Gln Val Ser Leu Pro Pro Leu
   850 855 860
   Asn Val Arg Gly Gly Arg Asp Ala Ile Ile Leu Leu Met Cys Ala Val
   865 870 875 880
   His Pro Glu Leu Ile Phe Asp Ile Thr Lys Leu Leu Leu Ser Ile Leu
   885 890 895
   Gly Pro Leu Met Val Leu Gln Ala Ser Leu Ile Arg Val Pro Tyr Phe
   900 905 910
   Val Arg Ala Gln Gly Leu Ile Arg Ala Cys Met Leu Val Arg Lys Ala
   915 920 925
   Ala Gly Gly His Tyr Val Gln Met Ala Phe Val Lys Leu Ala Ala Leu
   930 935 940
   Thr Gly Thr Tyr Val Tyr Asp His Leu Thr Pro Leu Gln Asp Trp Ala
   945 950 955 960
   His Val Gly Leu Arg Asp Leu Ala Val Ala Val Glu Pro Val Val Phe
   965 970 975
   Ser Ala Met Glu Thr Lys Val Ile Thr Trp Gly Ala Asp Thr Ala Ala
   980 985 990
   Cys Gly Asp Ile Ile Ser Gly Leu Pro Val Ser Ala Arg Arg Gly Lys
   995 1000 1005
   Glu Ile Leu Leu Gly Pro Ala Asp Ser Phe Glu Gly Gln Gly Trp
   1010 1015 1020
   Arg Leu Leu Ala Pro Ile Thr Ala Tyr Ser Gln Gln Thr Arg Gly
   1025 1030 1035
   Leu Leu Gly Cys Ile Ile Thr Ser Leu Thr Gly Arg Asp Lys Asn
   1040 1045 1050
   Gln Val Glu Gly Glu Val Gln Val Val Ser Thr Ala Lys Gln Ser
   1055 1060 1065
   Phe Leu Ala Thr Cys Val Asn Gly Ala Cys Trp Thr Val Phe His
   1070 1075 1080
   Gly Ala Gly Ser Lys Thr Leu Ala Ala Ala Lys Gly Pro Ile Thr
   1085 1090 1095
   Gln Met Tyr Thr Asn Val Asp Gln Asp Leu Val Gly Trp Pro Ala
   1100 1105 1110
   Pro Pro Gly Ala Arg Ser Leu Thr Pro Cys Thr Cys Gly Ser Ser
   1115 1120 1125
   Asp Leu Tyr Leu Val Thr Arg His Ala Asp Val Ile Pro Val Arg
   1130 1135 1140
   Arg Arg Gly Asp Ser Arg Gly Ser Leu Leu Ser Pro Arg Pro Ile
   1145 1150 1155
   Ser Tyr Leu Lys Gly Ser Ser Gly Gly Pro Leu Leu Cys Pro Ser
   1160 1165 1170
   Gly His Val Val Gly Ile Phe Arg Ala Ala Val Cys Thr Arg Gly
   1175 1180 1185
   Val Ala Lys Ala Val Asp Phe Ile Pro Val Glu Ser Met Glu Thr
   1190 1195 1200
   Thr Met Arg Ser Pro Val Phe Thr Asp Asn Ser Thr Pro Pro Ala
   1205 1210 1215
   Val Pro Gln Thr Phe Gln Val Ala His Leu His Ala Pro Thr Gly
   1220 1225 1230
   Ser Gly Lys Ser Thr Lys Val Pro Ala Ala Tyr Ala Ala Gln Gly
   1235 1240 1245
   Tyr Met Val Leu Val Leu Asn Pro Ser Val Ala Ala Thr Leu Gly
   1250 1255 1260
   Phe Gly Ala Tyr Met Ser Lys Ala His Gly Ile Asp Pro Asn Ile
   1265 1270 1275
   Arg Thr Gly Val Arg Thr Ile Thr Thr Gly Ala Pro Ile Thr Tyr
   1280 1285 1290
   Ser Thr Tyr Gly Lys Phe Leu Ala Asp Gly Gly Cys Ser Gly Gly
   1295 1300 1305
   Ala Tyr Asp Ile Ile Ile Cys Asp Glu Cys His Ser Thr Asp Ser
   1310 1315 1320
   Thr Ser Ile Leu Gly Ile Gly Thr Val Leu Asp Gln Ala Glu Thr
   1325 1330 1335
   Val Gly Ala Arg Phe Val Val Leu Ala Thr Ala Thr Pro Pro Gly
   1340 1345 1350
   Ser Ile Thr Phe Pro His Pro Asn Ile Glu Glu Val Pro Leu Ala
   1355 1360 1365
   Asn Thr Gly Glu Ile Pro Phe Tyr Ala Lys Thr Ile Pro Ile Glu
   1370 1375 1380
   Val Ile Arg Gly Gly Arg His Leu Ile Phe Cys His Ser Lys Lys
   1385 1390 1395
   Lys Cys Asp Glu Leu Pro Ala Lys Leu Ser Ala Leu Gly Leu Asn
   1400 1405 1410
   Ala Val Ala Tyr Tyr Arg Gly Leu Asp Val Ser Val Ile Pro Ala
   1415 1420 1425
   Ser Gly Asp Val Val Val Val Ala Thr Asp Ala Leu Met Thr Gly
   1430 1435 1440
   Phe Thr Gly Asp Phe Asp Ser Val Ile Asp Cys Asn Thr Cys Val
   1445 1450 1455
   Thr Gln Thr Val Asp Phe Ser Leu Asp Pro Thr Phe Thr Ile Glu
   1460 1465 1470
   Thr Thr Thr Val Pro Gln Asp Ala Val Ser Arg Thr Gln Arg Arg
   1475 1480 1485
   Gly Arg Thr Gly Arg Gly Arg Arg Gly Ile Tyr Arg Phe Val Thr
   1490 1495 1500
   Pro Gly Glu Arg Pro Ser Ala Met Phe Asp Ser Ser Val Leu Cys
   1505 1510 1515
   Glu Cys Tyr Asp Ala Gly Cys Ala Trp Tyr Glu Leu Thr Pro Ala
   1520 1525 1530
   Glu Thr Ser Val Arg Leu Arg Ala Tyr Leu Asn Thr Pro Gly Leu
   1535 1540 1545
   Pro Val Cys Gln Asp His Leu Glu Phe Trp Glu Ser Val Phe Thr
   1550 1555 1560
   Gly Leu Thr His Ile Asp Ala His Phe Leu Ser Gln Thr Lys Gln
   1565 1570 1575
   Ala Gly Asp Asn Phe Pro Tyr Leu Val Ala Tyr Gln Ala Thr Val
   1580 1585 1590
   Cys Ala Arg Ala Lys Ala Pro Pro Pro Ser Trp Asp Gln Met Trp
   1595 1600 1605
   Lys Cys Leu Ile Arg Leu Lys Pro Thr Leu His Gly Pro Thr Pro
   1610 1615 1620
   Leu Leu Tyr Arg Leu Gly Ala Val Gln Asn Glu Val Thr Leu Thr
   1625 1630 1635
   His Pro Ile Thr Lys Tyr Ile Met Ala Cys Met Ser Ala Asp Leu
   1640 1645 1650
   Glu Val Val Thr Ser Thr Trp Val Leu Val Gly Gly Val Leu Ala
   1655 1660 1665
   Ala Leu Ala Ala Tyr Cys Leu Thr Thr Gly Ser Val Val Ile Val
   1670 1675 1680
   Gly Arg Ile Ile Leu Ser Gly Arg Pro Ala Val Ile Pro Asp Arg
   1685 1690 1695
   Glu Val Leu Tyr Gln Glu Phe Asp Glu Met Glu Glu Cys Ala Ser
   1700 1705 1710
   His Leu Pro Tyr Ile Glu Gln Gly Met Gln Leu Ala Glu Gln Phe
   1715 1720 1725
   Lys Gln Lys Ala Leu Gly Leu Leu Gln Thr Ala Thr Lys Gln Ala
   1730 1735 1740
   Glu Ala Ala Ala Pro Val Val Glu Ser Lys Trp Arg Ala Leu Glu
   1745 1750 1755
   Thr Phe Trp Ala Lys His Met Trp Asn Phe Ile Ser Gly Ile Gln
   1760 1765 1770
   Tyr Leu Ala Gly Leu Ser Thr Leu Pro Gly Asn Pro Ala Ile Ala
   1775 1780 1785
   Ser Leu Met Ala Phe Thr Ala Ser Ile Thr Ser Pro Leu Ala Thr
   1790 1795 1800
   Gln Tyr Thr Leu Leu Phe Asn Ile Leu Gly Gly Trp Val Ala Ala
   1805 1810 1815
   Gln Leu Ala Pro Pro Ser Ala Ala Ser Ala Phe Val Gly Ala Gly
   1820 1825 1830
   Ile Ala Gly Ala Ala Val Gly Ser Ile Gly Leu Gly Lys Val Leu
   1835 1840 1845
   Val Asp Ile Leu Ala Gly Tyr Gly Ala Gly Val Ala Gly Ala Leu
   1850 1855 1860
   Val Ala Phe Lys Val Met Ser Gly Asp Met Pro Ser Thr Glu Asp
   1865 1870 1875
   Leu Val Asn Leu Leu Pro Ala Ile Leu Ser Pro Gly Ala Leu Val
   1880 1885 1890
   Val Gly Val Val Cys Ala Ala Ile Leu Arg Arg His Val Gly Pro
   1895 1900 1905
   Gly Glu Gly Ala Val Gln Trp Met Asn Arg Leu Ile Ala Phe Ala
   1910 1915 1920
   Ser Arg Gly Asn His Val Ser Pro Thr His Tyr Val Pro Glu Ser
   1925 1930 1935
   Asp Ala Ala Ala Arg Val Thr Gln Ile Leu Ser Asn Leu Thr Ile
   1940 1945 1950
   Thr Gln Leu Leu Lys Arg Leu His Gln Trp Ile Asn Glu Asp Cys
   1955 1960 1965
   Ser Thr Pro Cys Ser Gly Ser Trp Leu Arg Asp Val Trp Asp Trp
   1970 1975 1980
   Ile Cys Thr Val Leu Ala Asp Phe Lys Thr Trp Leu Gln Ser Lys
   1985 1990 1995
   Leu Leu Pro Arg Leu Pro Gly Val Pro Phe Phe Ser Cys Gln Arg
   2000 2005 2010
   Gly Tyr Lys Gly Val Trp Arg Gly Asp Gly Ile Met Tyr Thr Thr
   2015 2020 2025
   Cys Pro Cys Gly Ala Gln Ile Thr Gly His Val Lys Asn Gly Ser
   2030 2035 2040
   Met Arg Ile Val Gly Pro Arg Thr Cys Ser Asn Thr Trp His Gly
   2045 2050 2055
   Thr Phe Pro Ile Asn Ala Tyr Thr Thr Gly Pro Cys Thr Pro Ser
   2060 2065 2070
   Pro Ala Pro Asn Tyr Ser Arg Ala Leu Trp Arg Val Ala Ala Glu
   2075 2080 2085
   Glu Tyr Val Glu Val Thr Arg Val Gly Asp Phe His Tyr Val Thr
   2090 2095 2100
   Gly Met Thr Thr Asp Asn Val Lys Cys Pro Cys Gln Val Pro Ala
   2105 2110 2115
   Pro Glu Phe Phe Thr Glu Leu Asp Gly Val Arg Leu His Arg Tyr
   2120 2125 2130
   Ala Pro Ala Cys Lys Pro Leu Leu Arg Asp Glu Val Thr Phe Gln
   2135 2140 2145
   Val Gly Leu Asn Gln Tyr Thr Val Gly Ser Gln Leu Pro Cys Glu
   2150 2155 2160
   Pro Glu Pro Asp Val Thr Val Val Thr Ser Met Leu Thr Asp Pro
   2165 2170 2175
   Ser His Ile Thr Ala Glu Ala Ala Arg Arg Arg Leu Ala Arg Gly
   2180 2185 2190
   Ser Pro Pro Ser Leu Ala Gly Ser Ser Ala Ser Gln Leu Ser Ala
   2195 2200 2205
   Leu Ser Leu Lys Ala Thr Cys Thr Thr His His Gly Ala Pro Asp
   2210 2215 2220
   Thr Asp Leu Ile Glu Ala Asn Leu Leu Trp Arg Gln Glu Met Gly
   2225 2230 2235
   Gly Asn Ile Thr Arg Val Glu Ser Glu Asn Lys Ile Val Ile Leu
   2240 2245 2250
   Asp Ser Phe Glu Pro Leu Arg Ala Glu Glu Asp Glu Arg Glu Val
   2255 2260 2265
   Ser Ala Ala Ala Glu Ile Leu Arg Lys Thr Arg Lys Phe Pro Ala
   2270 2275 2280
   Ala Met Pro Val Trp Ala Arg Pro Asp Tyr Asn Pro Pro Leu Leu
   2285 2290 2295
   Glu Ser Trp Lys Asn Pro Asp Tyr Val Pro Pro Val Val His Gly
   2300 2305 2310
   Cys Pro Leu Pro Pro Thr Lys Ala Pro Pro Ile Pro Pro Pro Arg
   2315 2320 2325
   Arg Lys Arg Thr Val Val Leu Thr Glu Ser Thr Val Ser Ser Ala
   2330 2335 2340
   Leu Ala Glu Leu Ala Thr Lys Thr Phe Gly Gly Ser Gly Ser Ser
   2345 2350 2355
   Ala Val Asp Ser Gly Thr Ala Thr Gly Pro Pro Asp Gln Ala Ser
   2360 2365 2370
   Ala Glu Gly Asp Ala Gly Ser Asp Ala Glu Ser Tyr Ser Ser Met
   2375 2380 2385
   Pro Pro Leu Glu Gly Glu Pro Gly Asp Pro Asp Leu Ser Asp Gly
   2390 2395 2400
   Ser Trp Ser Thr Val Ser Glu Glu Ala Ser Glu Asp Val Val Cys
   2405 2410 2415
   Cys Ser Met Ser Tyr Thr Trp Thr Gly Ala Leu Ile Thr Pro Cys
   2420 2425 2430
   Ala Ala Glu Glu Ser Lys Leu Pro Ile Asn Ala Leu Ser Asn Pro
   2435 2440 2445
   Leu Leu Arg His His Asn Met Val Tyr Ser Thr Thr Ser Arg Ser
   2450 2455 2460
   Ala Ser Leu Arg Gln Lys Lys Val Thr Phe Asp Arg Met Gln Val
   2465 2470 2475
   Leu Asp Asp His Tyr Arg Asp Val Leu Lys Glu Met Lys Ala Lys
   2480 2485 2490
   Ala Ser Thr Val Lys Ala Lys Leu Leu Ser Val Glu Glu Ala Cys
   2495 2500 2505
   Lys Leu Thr Pro Pro His Ser Ala Lys Ser Lys Phe Gly Tyr Gly
   2510 2515 2520
   Ala Lys Asp Val Arg Ser Leu Ser Ser Arg Ala Val Asn His Ile
   2525 2530 2535
   Arg Ser Val Trp Lys Asp Leu Leu Glu Asp Thr Asp Thr Pro Ile
   2540 2545 2550
   Gln Thr Thr Ile Met Ala Lys Asn Glu Val Phe Cys Val Gln Pro
   2555 2560 2565
   Glu Lys Gly Gly Arg Lys Pro Ala Arg Leu Ile Val Phe Pro Asp
   2570 2575 2580
   Leu Gly Val Arg Val Cys Glu Lys Met Ala Leu Tyr Asp Val Val
   2585 2590 2595
   Ser Thr Leu Pro Gln Ala Val Met Gly Ser Ser Tyr Gly Phe Gln
   2600 2605 2610
   Tyr Ser Pro Lys Gln Arg Val Glu Phe Leu Val Asn Thr Trp Lys
   2615 2620 2625
   Ala Lys Lys Cys Pro Met Gly Phe Ser Tyr Asp Thr Arg Cys Phe
   2630 2635 2640
   Asp Ser Thr Val Thr Glu Asn Asp Ile Arg Val Glu Glu Ser Ile
   2645 2650 2655
   Tyr Gln Cys Cys Asp Leu Ala Pro Glu Ala Arg Gln Ala Ile Arg
   2660 2665 2670
   Ser Leu Thr Glu Arg Leu Tyr Ile Gly Gly Pro Met Thr Asn Ser
   2675 2680 2685
   Lys Gly Gln Asn Cys Gly Tyr Arg Arg Cys Arg Ala Ser Gly Val
   2690 2695 2700
   Leu Thr Thr Ser Cys Gly Asn Thr Leu Thr Cys Tyr Leu Lys Ala
   2705 2710 2715
   Ala Ala Ala Cys Arg Ala Ala Lys Leu Gln Asp Cys Thr Met Leu
   2720 2725 2730
   Val Cys Gly Asp Asp Leu Val Val Ile Cys Asp Ser Ala Gly Thr
   2735 2740 2745
   Gln Glu Asp Ala Ala Ser Leu Arg Val Phe Thr Glu Ala Met Thr
   2750 2755 2760
   Arg Tyr Ser Ala Pro Pro Gly Asp Pro Pro Gln Pro Glu Tyr Asp
   2765 2770 2775
   Leu Glu Leu Ile Thr Ser Cys Ser Ser Asn Val Ser Val Ala His
   2780 2785 2790
   Asp Ala Ser Gly Lys Arg Val Tyr Tyr Leu Thr Arg Asp Pro Thr
   2795 2800 2805
   Thr Pro Leu Ala Arg Ala Ala Trp Glu Thr Ala Arg His Thr Pro
   2810 2815 2820
   Val Asn Ser Trp Leu Gly Asn Ile Ile Met Tyr Ala Pro Thr Leu
   2825 2830 2835
   Trp Ala Arg Met Ile Leu Met Thr His Phe Phe Ser Ile Leu Leu
   2840 2845 2850
   Ala Gln Glu Gln Leu Glu Lys Ala Leu Asp Cys Gln Ile Tyr Gly
   2855 2860 2865
   Ala Thr Tyr Ser Ile Glu Pro Leu Asp Leu Pro Gln Ile Ile Gln
   2870 2875 2880
   Arg Leu His Gly Leu Ser Ala Phe Ser Leu His Ser Tyr Ser Pro
   2885 2890 2895
   Gly Glu Ile Asn Arg Val Ala Ser Cys Leu Arg Lys Leu Gly Val
   2900 2905 2910
   Pro Pro Leu Arg Val Trp Arg His Arg Ala Arg Ser Val Arg Ala
   2915 2920 2925
   Lys Leu Leu Ser Gln Gly Gly Arg Ala Ala Thr Cys Gly Lys Tyr
   2930 2935 2940
   Leu Phe Asn Trp Ala Val Lys Thr Lys Leu Lys Leu Thr Pro Ile
   2945 2950 2955
   Pro Glu Ala Ser Gln Leu Asp Leu Ser Gly Trp Phe Val Ala Gly
   2960 2965 2970
   Tyr Ser Gly Gly Asp Ile Tyr His Ser Leu Ser Arg Ala Arg Pro
   2975 2980 2985
   Arg Trp Phe Met Trp Cys Leu Leu Leu Leu Ser Val Gly Val Gly
   2990 2995 3000
   Ile Tyr Leu Leu Pro Asn Arg
   3005 3010
<210> 186
   <211> 3010
   <212> PRT
   <213> Hepatitis C virus
<400> 186
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg His Pro Glu Gly Arg Ala Trp Ala Gln Pro Gly
   65 70 75 80
   Tyr Pro Trp Pro Leu Tyr Gly Asn Glu Gly Met Gly Trp Ala Gly Trp
   85 90 95
   Leu Leu Ser Pro Arg Gly Ser Arg Pro Ser Trp Gly Pro Thr Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
   130 135 140
   Gly Gly Ala Ala Arg Ala Leu Ala His Gly Val Arg Val Leu Glu Asp
   145 150 155 160
   Gly Val Asn Tyr Ala Thr Gly Asn Leu Pro Gly Cys Ser Phe Ser Ile
   165 170 175
   Phe Leu Leu Ala Leu Leu Ser Cys Leu Thr Ile Pro Ala Ser Ala Tyr
   180 185 190
   Glu Val Arg Asn Val Ser Gly Ile Tyr His Val Thr Asn Asp Cys Ser
   195 200 205
   Asn Ser Ser Ile Val Tyr Glu Ala Ala Asp Val Ile Met His Ala Pro
   210 215 220
   Gly Cys Val Pro Cys Val Arg Glu Asn Asn Ser Ser Arg Cys Trp Val
   225 230 235 240
   Ala Leu Thr Pro Thr Leu Ala Ala Arg Asn Ala Ser Val Pro Thr Thr
   245 250 255
   Thr Leu Arg Arg His Val Asp Leu Leu Val Gly Thr Ala Ala Phe Cys
   260 265 270
   Ser Ala Met Tyr Val Gly Asp Leu Cys Gly Ser Val Phe Leu Ile Ser
   275 280 285
   Gln Leu Phe Thr Phe Ser Pro Arg Arg His Glu Thr Val Gln Asp Cys
   290 295 300
   Asn Cys Ser Ile Tyr Pro Gly His Val Ser Gly His Arg Met Ala Trp
   305 310 315 320
   Asp Met Met Met Asn Trp Ser Pro Thr Ala Ala Leu Val Val Ser Gln
   325 330 335
   Leu Leu Arg Ile Pro Gln Ala Val Met Asp Met Val Ala Gly Ala His
   340 345 350
   Trp Gly Val Leu Ala Gly Leu Ala Tyr Tyr Ser Met Val Gly Asn Trp
   355 360 365
   Ala Lys Val Leu Ile Val Met Leu Leu Phe Ala Gly Val Asp Gly His
   370 375 380
   Thr Arg Val Thr Gly Gly Val Gln Gly His Val Thr Ser Thr Leu Thr
   385 390 395 400
   Ser Leu Phe Arg Pro Gly Ala Ser Gln Lys Ile Gln Leu Val Asn Thr
   405 410 415
   Asn Gly Ser Trp His Ile Asn Arg Thr Ala Leu Asn Cys Asn Asp Ser
   420 425 430
   Leu Lys Thr Gly Phe Leu Ala Ala Leu Phe Tyr Thr His Lys Phe Asn
   435 440 445
   Ala Ser Gly Cys Pro Glu Arg Met Ala Ser Cys Arg Ser Ile Asp Lys
   450 455 460
   Phe Asp Gln Gly Trp Gly Pro Ile Thr Tyr Ala Gln Pro Asp Asn Ser
   465 470 475 480
   Asp Gln Arg Pro Tyr Cys Trp His Tyr Ala Pro Arg Gln Cys Gly Ile
   485 490 495
   Val Pro Ala Ser Gln Val Cys Gly Pro Val Tyr Cys Phe Thr Pro Ser
   500 505 510
   Pro Val Val Val Gly Thr Thr Asp Arg Phe Gly Ala Pro Thr Tyr Asn
   515 520 525
   Trp Gly Asp Asn Glu Thr Asp Val Leu Leu Leu Asn Asn Thr Arg Pro
   530 535 540
   Pro His Gly Asn Trp Phe Gly Cys Thr Trp Met Asn Ser Thr Gly Phe
   545 550 555 560
   Thr Lys Thr Cys Gly Gly Pro Pro Cys Asn Ile Arg Gly Val Gly Asn
   565 570 575
   Asn Thr Leu Thr Cys Pro Thr Asp Cys Phe Arg Lys His Pro Asp Ala
   580 585 590
   Thr Tyr Thr Lys Cys Gly Ser Gly Pro Trp Leu Thr Pro Arg Cys Leu
   595 600 605
   Val Asp Tyr Pro Tyr Arg Leu Trp His Tyr Pro Cys Thr Val Asn Phe
   610 615 620
   Thr Ile Phe Lys Val Arg Met Tyr Val Gly Gly Val Glu His Arg Leu
   625 630 635 640
   Asp Ala Ala Cys Asn Trp Thr Arg Gly Glu Arg Cys Asp Leu Glu Asp
   645 650 655
   Arg Asp Arg Ala Glu Leu Ser Pro Leu Leu Leu Ser Thr Thr Glu Trp
   660 665 670
   Gln Ile Leu Pro Cys Ser Tyr Thr Thr Leu Pro Ala Leu Ser Thr Gly
   675 680 685
   Leu Ile His Leu His Gln Asn Ile Val Asp Ile Gln Tyr Leu Tyr Gly
   690 695 700
   Ile Gly Ser Ala Val Val Ser Ile Ala Ile Lys Trp Glu Tyr Val Val
   705 710 715 720
   Leu Leu Phe Leu Leu Leu Ala Asp Ala Arg Val Cys Ala Cys Leu Trp
   725 730 735
   Met Met Leu Leu Ile Ala Gln Ala Glu Ala Ala Leu Glu Asn Leu Val
   740 745 750
   Val Leu Asn Ala Ala Ser Val Ala Gly Ala His Gly Ile Leu Pro Phe
   755 760 765
   Phe Met Phe Phe Cys Ala Ala Trp Tyr Met Lys Gly Arg Leu Val Pro
   770 775 780
   Gly Ala Ala Tyr Ala Phe Tyr Gly Val Trp Pro Leu Leu Leu Leu Leu
   785 790 795 800
   Leu Ala Leu Pro Pro Arg Ala Tyr Ala Met Asp Arg Glu Met Ala Ala
   805 810 815
   Ser Cys Gly Gly Gly Val Phe Val Gly Leu Ala Leu Leu Thr Leu Ser
   820 825 830
   Pro Tyr Cys Lys Val Phe Leu Ala Arg Leu Ile Trp Trp Leu Gln Tyr
   835 840 845
   Phe Ile Thr Lys Ala Glu Ala His Leu Gln Val Trp Val Pro Pro Leu
   850 855 860
   Asn Val Arg Ala Gly Arg Asp Ala Ile Ile Leu Leu Met Cys Ala Val
   865 870 875 880
   His Pro Glu Leu Ile Phe Asp Ile Thr Lys Leu Leu Leu Ser Ile Leu
   885 890 895
   Gly Pro Leu Met Val Leu Gln Ala Ser Leu Ile Arg Val Pro Tyr Phe
   900 905 910
   Val Arg Ala Gln Gly Leu Ile Arg Ala Cys Thr Leu Val Arg Lys Ala
   915 920 925
   Ala Gly Gly His Tyr Val Gln Met Ala Phe Val Lys Leu Ala Ala Leu
   930 935 940
   Thr Gly Thr Tyr Val Tyr Asp His Leu Thr Pro Leu Gln Asp Trp Ala
   945 950 955 960
   His Val Gly Leu Arg Asp Leu Ala Val Ala Val Glu Pro Val Val Phe
   965 970 975
   Ser Ala Met Glu Thr Lys Val Ile Thr Trp Gly Ala Asp Thr Ala Ala
   980 985 990
   Cys Gly Asp Ile Ile Ser Gly Leu Pro Val Ser Ala Arg Arg Gly Lys
   995 1000 1005
   Glu Ile Leu Leu Gly Pro Ala Asp Ser Phe Glu Gly Gln Gly Trp
   1010 1015 1020
   Arg Leu Leu Ala Pro Ile Thr Ala Tyr Ser Gln Gln Thr Arg Gly
   1025 1030 1035
   Leu Leu Gly Cys Ile Ile Thr Ser Leu Thr Gly Arg Asp Lys Asn
   1040 1045 1050
   Gln Val Glu Gly Glu Val Gln Val Val Ser Thr Ala Thr Gln Ser
   1055 1060 1065
   Phe Leu Ala Thr Cys Val Asn Gly Ala Cys Trp Thr Val Phe His
   1070 1075 1080
   Gly Ala Gly Ser Lys Thr Leu Ala Gly Pro Lys Gly Pro Ile Thr
   1085 1090 1095
   Gln Met Tyr Thr Asn Val Asp Gln Asp Leu Val Gly Trp Pro Ala
   1100 1105 1110
   Pro Pro Gly Ala Arg Ser Leu Thr Pro Cys Thr Cys Gly Ser Ser
   1115 1120 1125
   Asp Leu Tyr Leu Val Thr Arg His Ala Asp Val Ile Pro Val Arg
   1130 1135 1140
   Arg Arg Gly Asp Thr Arg Gly Ser Leu Leu Ser Pro Arg Pro Ile
   1145 1150 1155
   Ser Tyr Leu Lys Gly Ser Ser Gly Gly Pro Leu Leu Cys Pro Ser
   1160 1165 1170
   Gly His Val Val Gly Ile Phe Arg Ala Ala Val Cys Thr Arg Gly
   1175 1180 1185
   Val Ala Lys Ala Val Asp Phe Ile Pro Val Glu Ser Met Glu Thr
   1190 1195 1200
   Thr Met Arg Ser Pro Val Phe Thr Asp Asn Ser Thr Pro Pro Ala
   1205 1210 1215
   Val Pro Gln Thr Phe Gln Val Ala His Leu His Ala Pro Thr Gly
   1220 1225 1230
   Ser Gly Lys Ser Thr Lys Val Pro Ala Ala Tyr Ala Ala Gln Gly
   1235 1240 1245
   Tyr Met Val Leu Val Leu Asn Pro Ser Val Ala Ala Thr Leu Gly
   1250 1255 1260
   Phe Gly Ala Tyr Met Ser Lys Ala His Gly Ile Asp Pro Asn Ile
   1265 1270 1275
   Arg Thr Gly Val Arg Thr Ile Thr Thr Gly Ala Pro Ile Thr Tyr
   1280 1285 1290
   Ser Thr Tyr Gly Lys Phe Leu Ala Asp Gly Gly Cys Ser Gly Gly
   1295 1300 1305
   Ala Tyr Asp Ile Ile Ile Cys Asp Glu Cys His Ser Thr Asp Ser
   1310 1315 1320
   Thr Ser Ile Leu Gly Ile Gly Thr Val Leu Asp Gln Ala Glu Thr
   1325 1330 1335
   Ala Gly Ala Arg Leu Val Val Leu Ala Thr Ala Thr Pro Pro Gly
   1340 1345 1350
   Ser Val Thr Phe Pro His Pro Asn Ile Glu Glu Val Ala Leu Gly
   1355 1360 1365
   Asn Thr Gly Glu Ile Pro Phe Tyr Gly Lys Ala Ile Pro Ile Glu
   1370 1375 1380
   Val Ile Lys Gly Gly Arg His Leu Ile Phe Cys His Ser Lys Lys
   1385 1390 1395
   Lys Cys Asp Glu Leu Ala Ala Lys Leu Ser Pro Leu Gly Leu Asn
   1400 1405 1410
   Ala Val Ala Tyr Tyr Arg Gly Leu Asp Val Ser Val Ile Pro Ala
   1415 1420 1425
   Ser Gly Asp Val Val Val Val Ala Thr Asp Ala Leu Met Thr Gly
   1430 1435 1440
   Phe Thr Gly Asp Phe Asp Ser Val Ile Asp Cys Asn Thr Cys Val
   1445 1450 1455
   Thr Gln Thr Val Asp Phe Ser Leu Asp Pro Thr Phe Thr Ile Glu
   1460 1465 1470
   Thr Thr Thr Val Pro Gln Asp Ala Val Ser Arg Thr Gln Arg Arg
   1475 1480 1485
   Gly Arg Thr Gly Arg Gly Arg Arg Gly Ile Tyr Arg Phe Val Thr
   1490 1495 1500
   Pro Gly Glu Arg Pro Ser Gly Met Phe Asp Ser Ser Val Leu Cys
   1505 1510 1515
   Glu Cys Tyr Asp Ala Gly Cys Ala Trp Tyr Glu Leu Thr Pro Ala
   1520 1525 1530
   Glu Thr Ser Val Arg Leu Arg Ala Tyr Leu Asn Thr Pro Gly Leu
   1535 1540 1545
   Pro Val Cys Gln Asp His Leu Glu Phe Trp Glu Ser Val Phe Thr
   1550 1555 1560
   Gly Leu Thr His Ile Asp Ala His Phe Leu Ser Gln Thr Lys Gln
   1565 1570 1575
   Ala Gly Asp Asn Phe Pro Tyr Leu Val Ala Tyr Gln Ala Thr Val
   1580 1585 1590
   Cys Ala Arg Ala Lys Ala Pro Pro Pro Ser Trp Asp Gln Met Trp
   1595 1600 1605
   Lys Cys Leu Ile Arg Leu Lys Pro Thr Leu His Gly Pro Thr Pro
   1610 1615 1620
   Leu Leu Tyr Arg Leu Gly Ala Val Gln Asn Glu Val Thr Leu Thr
   1625 1630 1635
   His Pro Ile Thr Lys Phe Ile Met Ala Cys Met Ser Ala Asp Leu
   1640 1645 1650
   Glu Val Val Thr Ser Thr Trp Val Leu Val Gly Gly Val Leu Ala
   1655 1660 1665
   Ala Leu Ala Ala Tyr Cys Leu Thr Thr Gly Ser Val Val Ile Val
   1670 1675 1680
   Gly Arg Ile Ile Leu Ser Gly Arg Pro Ala Val Ile Pro Asp Arg
   1685 1690 1695
   Glu Val Leu Tyr Gln Glu Phe Asp Glu Met Glu Glu Cys Ala Ser
   1700 1705 1710
   His Leu Pro Tyr Ile Glu Gln Gly Met Gln Leu Ala Glu Gln Phe
   1715 1720 1725
   Lys Gln Lys Ala Leu Gly Leu Leu Gln Thr Ala Thr Lys Gln Ala
   1730 1735 1740
   Glu Ala Ala Ala Pro Val Val Glu Ser Lys Trp Arg Ala Leu Glu
   1745 1750 1755
   Thr Phe Trp Ala Lys His Met Trp Asn Phe Ile Ser Gly Ile Gln
   1760 1765 1770
   Tyr Leu Ala Gly Leu Ser Thr Leu Pro Gly Asn Pro Ala Ile Ala
   1775 1780 1785
   Ser Leu Met Ala Phe Thr Ala Ser Ile Thr Ser Pro Leu Ala Thr
   1790 1795 1800
   Gln Tyr Thr Leu Leu Phe Asn Ile Leu Gly Gly Trp Val Ala Ala
   1805 1810 1815
   Gln Leu Ala Pro Pro Ser Ala Ala Ser Ala Phe Val Gly Ala Gly
   1820 1825 1830
   Ile Ala Gly Ala Ala Val Gly Ser Ile Gly Leu Gly Lys Val Leu
   1835 1840 1845
   Val Asp Ile Leu Ala Gly Tyr Gly Ala Gly Val Ala Gly Ala Leu
   1850 1855 1860
   Val Ala Phe Lys Val Met Ser Gly Asp Met Pro Ser Thr Glu Asp
   1865 1870 1875
   Leu Val Asn Leu Leu Pro Ala Ile Leu Ser Pro Gly Ala Leu Val
   1880 1885 1890
   Val Gly Val Val Cys Ala Ala Ile Leu Arg Arg His Val Gly Pro
   1895 1900 1905
   Gly Glu Gly Ala Val Gln Trp Met Asn Arg Leu Ile Ala Phe Ala
   1910 1915 1920
   Ser Arg Gly Asn His Val Ser Pro Thr His Tyr Val Pro Glu Ser
   1925 1930 1935
   Asp Ala Ala Ala Arg Val Thr Gln Ile Leu Ser Asn Leu Thr Ile
   1940 1945 1950
   Thr Gln Leu Leu Lys Arg Leu His Gln Trp Ile Asn Glu Asp Cys
   1955 1960 1965
   Ser Thr Pro Cys Ser Gly Ser Trp Leu Arg Asp Val Trp Asp Trp
   1970 1975 1980
   Ile Cys Thr Val Leu Ala Asp Phe Lys Thr Trp Leu Gln Ser Lys
   1985 1990 1995
   Leu Leu Pro Arg Leu Pro Gly Val Pro Phe Phe Ser Cys Gln Arg
   2000 2005 2010
   Gly Tyr Lys Gly Val Trp Arg Gly Asp Gly Ile Met Tyr Thr Thr
   2015 2020 2025
   Cys Pro Cys Gly Ala Gln Ile Thr Gly His Val Lys Asn Gly Ser
   2030 2035 2040
   Met Arg Ile Val Gly Pro Arg Thr Cys Ser Asn Thr Trp His Gly
   2045 2050 2055
   Thr Phe Pro Ile Asn Ala Tyr Thr Thr Gly Pro Cys Thr Pro Ser
   2060 2065 2070
   Pro Ala Pro Asn Tyr Ser Arg Ala Leu Trp Arg Val Ala Ala Glu
   2075 2080 2085
   Glu Tyr Val Glu Val Thr Arg Val Gly Asp Phe His Tyr Val Thr
   2090 2095 2100
   Gly Met Thr Thr Asp Asn Val Lys Cys Pro Cys Gln Val Pro Ala
   2105 2110 2115
   Pro Glu Phe Phe Thr Glu Leu Asp Gly Val Arg Leu His Arg Tyr
   2120 2125 2130
   Ala Pro Ala Cys Lys Pro Leu Leu Arg Asp Glu Val Thr Phe Gln
   2135 2140 2145
   Val Gly Leu Asn Gln Tyr Thr Val Gly Ser Gln Leu Pro Cys Glu
   2150 2155 2160
   Pro Glu Pro Asp Val Thr Val Val Thr Ser Met Leu Thr Asp Pro
   2165 2170 2175
   Ser His Ile Thr Ala Glu Ala Ala Arg Arg Arg Leu Ala Arg Gly
   2180 2185 2190
   Ser Pro Pro Ser Leu Ala Gly Ser Ser Ala Ser Gln Leu Ser Ala
   2195 2200 2205
   Pro Ser Leu Lys Ala Thr Cys Thr Thr His His Gly Ala Pro Asp
   2210 2215 2220
   Thr Asp Leu Ile Glu Ala Asn Leu Leu Trp Arg Gln Glu Met Gly
   2225 2230 2235
   Gly Asn Ile Thr Arg Val Glu Ser Glu Asn Lys Ile Val Ile Leu
   2240 2245 2250
   Asp Ser Phe Glu Pro Leu Arg Ala Glu Glu Asp Glu Arg Glu Val
   2255 2260 2265
   Ser Ala Ala Ala Glu Ile Leu Arg Lys Thr Arg Lys Phe Pro Ala
   2270 2275 2280
   Ala Met Pro Val Trp Ala Arg Pro Asp Tyr Asn Pro Pro Leu Leu
   2285 2290 2295
   Glu Ser Trp Lys Asn Pro Asp Tyr Val Pro Pro Val Val His Gly
   2300 2305 2310
   Cys Pro Leu Pro Pro Thr Lys Ala Pro Pro Ile Pro Pro Pro Arg
   2315 2320 2325
   Arg Lys Arg Thr Val Val Leu Thr Glu Ser Thr Val Ser Ser Ala
   2330 2335 2340
   Leu Ala Glu Leu Ala Thr Lys Thr Phe Gly Gly Ser Gly Ser Ser
   2345 2350 2355
   Ala Val Asp Ser Gly Thr Ala Thr Gly Pro Pro Asp Gln Ala Ser
   2360 2365 2370
   Ala Glu Gly Asp Ala Gly Ser Asp Ala Glu Ser Tyr Ser Ser Met
   2375 2380 2385
   Pro Pro Leu Glu Gly Glu Pro Gly Asp Pro Asp Leu Ser Asp Gly
   2390 2395 2400
   Ser Trp Ser Thr Val Ser Glu Glu Ala Ser Glu Asp Val Val Cys
   2405 2410 2415
   Cys Ser Met Ser Tyr Thr Trp Thr Gly Ala Leu Ile Thr Pro Cys
   2420 2425 2430
   Ala Ala Glu Glu Ser Lys Leu Pro Ile Asn Ala Leu Ser Asn Pro
   2435 2440 2445
   Leu Leu Arg His His Asn Met Val Tyr Ser Thr Thr Ser Arg Ser
   2450 2455 2460
   Ala Ser Leu Arg Gln Lys Lys Val Thr Phe Asp Arg Met Gln Val
   2465 2470 2475
   Leu Asp Asp His Tyr Arg Asp Val Leu Lys Glu Met Lys Ala Lys
   2480 2485 2490
   Ala Ser Thr Val Lys Ala Lys Leu Leu Ser Val Glu Glu Ala Cys
   2495 2500 2505
   Lys Leu Thr Pro Pro His Ser Ala Lys Ser Lys Phe Gly Tyr Gly
   2510 2515 2520
   Ala Lys Asp Val Arg Ser Leu Ser Ser Arg Ala Val Asn His Ile
   2525 2530 2535
   Arg Ser Val Trp Lys Asp Leu Leu Glu Asp Thr Asp Thr Pro Ile
   2540 2545 2550
   Gln Thr Thr Ile Met Ala Lys Asn Glu Val Phe Cys Val Gln Pro
   2555 2560 2565
   Glu Lys Gly Gly Arg Lys Pro Ala Arg Leu Ile Val Phe Pro Asp
   2570 2575 2580
   Leu Gly Val Arg Val Cys Glu Lys Met Ala Leu Tyr Asp Val Val
   2585 2590 2595
   Ser Thr Leu Pro Gln Ala Val Met Gly Ser Ser Tyr Gly Phe Gln
   2600 2605 2610
   Tyr Ser Pro Lys Gln Arg Val Glu Phe Leu Val Asn Thr Trp Lys
   2615 2620 2625
   Ala Lys Lys Cys Pro Met Gly Phe Ser Tyr Asp Thr Arg Cys Phe
   2630 2635 2640
   Asp Ser Thr Val Thr Glu Asn Asp Ile Arg Val Glu Glu Ser Ile
   2645 2650 2655
   Tyr Gln Cys Cys Asp Leu Ala Pro Glu Ala Arg Gln Ala Ile Arg
   2660 2665 2670
   Ser Leu Thr Glu Arg Leu Tyr Ile Gly Gly Pro Met Thr Asn Ser
   2675 2680 2685
   Lys Gly Gln Asn Cys Gly Tyr Arg Arg Cys Arg Ala Ser Gly Val
   2690 2695 2700
   Leu Thr Thr Ser Cys Gly Asn Thr Leu Thr Cys Tyr Leu Lys Ala
   2705 2710 2715
   Ala Ala Ala Cys Arg Ala Ala Lys Leu Gln Asp Cys Thr Met Leu
   2720 2725 2730
   Val Cys Gly Asp Asp Leu Val Val Ile Cys Asp Ser Ala Gly Thr
   2735 2740 2745
   Gln Glu Asp Ala Ala Ser Leu Arg Val Phe Thr Glu Ala Met Thr
   2750 2755 2760
   Arg Tyr Ser Ala Pro Pro Gly Asp Pro Pro Gln Pro Glu Tyr Asp
   2765 2770 2775
   Leu Glu Leu Ile Thr Ser Cys Ser Ser Asn Val Ser Val Ala His
   2780 2785 2790
   Asp Ala Ser Gly Lys Arg Val Tyr Tyr Leu Thr Arg Asp Pro Thr
   2795 2800 2805
   Thr Pro Leu Ala Arg Ala Ala Trp Glu Thr Ala Arg His Thr Pro
   2810 2815 2820
   Val Asn Ser Trp Leu Gly Asn Ile Ile Met Tyr Ala Pro Thr Leu
   2825 2830 2835
   Trp Ala Arg Met Ile Leu Met Thr His Phe Phe Ser Ile Leu Leu
   2840 2845 2850
   Ala Gln Glu Gln Leu Glu Lys Ala Leu Asp Cys Gln Ile Tyr Gly
   2855 2860 2865
   Ala Thr Tyr Ser Ile Glu Pro Leu Asp Leu Pro Gln Ile Ile Gln
   2870 2875 2880
   Arg Leu His Gly Leu Ser Ala Phe Ser Leu His Ser Tyr Ser Pro
   2885 2890 2895
   Gly Glu Ile Asn Arg Val Ala Ser Cys Leu Arg Lys Leu Gly Val
       2900                        2905                        2910
   Pro Pro Leu Arg Val Trp Arg His Arg Ala Arg Ser Val Arg Ala
       2915                        2920                        2925
   Lys Leu Leu Ser Gln Gly Gly Arg Ala Ala Thr Cys Gly Lys Tyr
       2930                        2935                        2940
   Leu Phe Asn Trp Ala Val Lys Thr Lys Leu Lys Leu Thr Pro Ile
       2945                        2950                        2955
   Pro Glu Ala Ser Gln Leu Asp Leu Ser Gly Trp Phe Val Ala Gly
       2960                        2965                        2970
   Tyr Ser Gly Gly Asp Ile Tyr His Ser Leu Ser Arg Ala Arg Pro
       2975                        2980                        2985
   Arg Trp Phe Met Trp Cys Leu Leu Leu Leu Ser Val Gly Val Gly
       2990                        2995                        3000
   Ile Tyr Leu Leu Pro Asn Arg
       3005                        3010